# EUROPÄISCHE PATENTSCHRIFT

(11) **EP 3 532 841 B1**
(45) Veröffentlichungstag und Bekanntmachung des Hinweises auf die Patenterteilung: **08.01.2020**
(21) Anmeldenummer: 17783386.0
(22) Anmeldetag: 28.07.2017
(51) Int. Cl.: G01N 33/28, B01L 3/00, G01N 21/01

(54) **PROBENAUFNAHMEELEMENT, ANALYSENSET UND VERFAHREN ZUR ANALYSE EINES LIQUIDS, INSBESONDERE EINER KÜHLSCHMIERSTOFFEMULSION**
SAMPLE RECEIVING ELEMENT, ANALYSES SET AND METHOD FOR ANALYZING A LIQUID, IN PARTICULAR A COOLING LUBRICANT EMULSION
ÉLÉMENT DE RÉCEPTION D'ÉCHANTILLONS, KIT D'ANALYSE ET PROCÉDÉ D'ANALYSE D'UN LIQUIDE, EN PARTICULIER D'UNE ÉMULSION D'HUILE DE REFROIDISSEMENT

(30) Priorität: 26.10.2016 EP 16002281
(43) Veröffentlichungstag der Anmeldung: 04.09.2019
(73) Patentinhaber: Fuchs Petrolub SE, 68169 Mannheim (DE)
(72) Erfinder: FUCHS, Christine, 40474 Düsseldorf (DE); THEIS, Heinz Gerhard, 67368 Westheim (DE)
(74) Vertreter: Mehl-Mikus, Claudia
(86) Internationale Anmeldenummer: PCT/EP2017/000917
(87) Internationale Veröffentlichungsnummer: WO 2018/077449

(56) Entgegenhaltungen:
- WO-A2-00/13002
- DE-T2- 69 634 490
- US-A1- 2011 201 099
- US-A1- 2013 330 245

## Beschreibung

Die Erfindung betrifft ein Probeaufnahmeelement für eine Liquidprobe, ein Analysevorrichtungs-Set für die simultane Analyse von drei oder mehr chemischen und physikalischen Parameter von Liquiden, das ein als Handgerät ausgebildetes Analysegerät und das Probeaufnahmeelement für die Liquidprobe umfasst, die Verwendung des Sets und ein Verfahren, für dessen Durchführung das Analysengerät unter Verwendung des Probeaufnahmeelements eingesetzt wird.

Aus dem Stand der Technik sind Messgeräte bekannt, mit denen verschiedene Parameter von Kühlschmierstoffen gemessen oder überprüft werden können. So sind Refraktometer bekannt, mit denen die Brechzahl des Kühlschmierstoffs ermittelt wird. Bei wassergemischten Kühlschmierstoffen kann aus der Brechzahl auf das Mischungsverhältnis geschlossen werden. Ferner sind Messgeräte zur Bestimmung der elektrischen Leitfähigkeit bekannt, bei denen der Widerstand über eine bestimmte Messstrecke ermittelt wird. Weiter gibt es Messgeräte zur Bestimmung des pH-Wertes der Kühlschmierstoffe. Im Wesentlichen werden dabei zwei Typen pH-Messgeräte eingesetzt: pH-Meter mit Elektrode und optochemische pH-Messgeräte.

Darüber hinaus gibt es Messgeräte, die nach vorheriger Auswahl der Messgröße unterschiedliche Parameter ermitteln können.

So offenbart die DE 10 2010 028 319 ein Verfahren zur Steuerung der Konzentration wassergemischter Kühlschmierstoffe, bei dem zur Bestimmung der Brechzahl des Kühlschmierstoffs ein Refraktometer eingesetzt und die elektrische Leitfähigkeit durch eine Widerstandsmessung, deren Kehrwert die Leitfähigkeit ergibt, erfasst wird. Weiter wird die Temperatur des Kühlschmierstoffes überwacht, um die aus Temperaturschwankungen entstehenden Änderungen der Daten zu berücksichtigen. Aus den gemessenen Größen werden Rückschlüsse über die Zusammensetzung des Kühlschmierstoffs gezogen und diese im Bedarfsfall angepasst.

DE 696 34 490 T2 offenbart eine scheibenförmige Mikrosystem-Plattform mit zwei flachen planaren Oberflächen als Probenaufnahmeelement für eine Liquidprobe. Die scheibenförmige Mikrosystem-Plattform weist Eingangstore für eine flüssige Probe, Flüssigkeitsmikrokanäle, Reaktions- und Detektionskammern auf, wobei zur Analyse der Liquidprobe mehrere Messstellen auf der Scheibe vorgesehen sind. Die durchführbaren Messungen umfassen Lumineszenzmessungen und Brechungsindexmessungen sowie elektrochemische Detektionsmethoden. Die zugehörige Analysenvorrichtung ist ähnlich einem CD-Player mit Elementen zum Drehen und Lesen der scheibenförmigen Mikrosystem-Plattform zur Steuerung der Funktionen ausgestattet. Nach Applikation des zu prüfenden Analyts in die Eingangstore wird die Mikrosystem-Plattform in die CD-Playervorrichtung eingesetzt, wobei der Flüssigkeitstransport durch die Mikrokanäle auf der Scheibe mittels zentripetaler Beschleunigung in der CD-Playervorrichtung und durch selektive Aktivierung von Ventilen auf der Scheibe erfolgt. Die Analyseresultate können gespeichert und/oder sofort dem Benutzer angezeigt werden.

Aus US 2011/201099 A1 ist eine Probenkassette mit Kanälen und Kammern bekannt, die Elektroden und Detektionsfenster für optische Messungen aufweisen kann. In den Kammern können Reagenzien wie Bindungsreagenzien, nachweisbare Markierungen, Probenaufbereitungsreagenzien, Waschlösungen, Puffer, etc. in flüssiger oder fester Form oder auf der Oberfläche von festen immobilisiert Phasenträger vorliegen. Das zugehörige Analysengerät, das nicht als Handgerät ausgebildet ist, weist für die vorzunehmenden Messungen entsprechende Detektoren und Mittel zum Aufnehmen der Kassette und zum Positionieren der Kassette sowie elektrische Systeme zum Kontaktieren der Elektroden der Kassette sowie Steuerungssysteme zum Erfassen, Verarbeiten und Speichern der Signale der Detektoren auf. Für Lumineszenzmessungen weist das Analysengerät einen lichtdicht geschlossenen Bereich auf. Zur Aufnahme und Positionierung des Probenaufnahmeelements weist das Analysengerät ein Kassettenfach auf, das über einen Führungsschlitten auf Schienen montiert ist, um eine motorgetriebene Bewegung des Fachs in den und aus dem lichtdicht geschlossenen Bereich zu ermöglichen.

US 2013/330245 A1 beschreibt ein Probenaufnahmeelement mit Kanälen und einer Detektionskammer für optische Messungen sowie Elektroden am Fluideinlass zur Messung des Widerstands der Probe, um bei der Probennahme ein genügendes Eintauchen des Probenaufnahmeelements in die zu prüfende Flüssigkeit zu signalisieren. Das Probenaufnahmeelement wird zur Probennahme in das als Handgerät ausgebildete zugehörige Analysengerät eingeführt, das zur Analyse mehrerer Parameter gleichzeitig mehrere Probenaufnahmeelemente aufnehmen kann und entsprechende optische Analysenvorrichtungen etc. aufweist. Bei der Probennahme wird eine Liquidprobe durch eine Pumpe der Analysenvorrichtung in das Probenaufnahmeelement gezogen.

Ausgehend von diesem Stand der Technik ist es Aufgabe der vorliegenden Erfindung, eine verbesserte Vorrichtung zu schaffen, mit der ein Liquid - wie ein Kühlschmiermittel - bei minimiertem Probenmengenaufwand zur Messung der Konzentration des Liquids oder seiner Komponenten und zur Messung oder Bestimmung von mehreren weiteren Parametern wie dem Brechungsindex und der Temperatur und optional noch weiteren Parametern, die die Beschaffenheit des Fluids kennzeichnen, mit einfachster Handhabung präpariert werden kann.

Diese Aufgabe wird mit Hilfe eines Probeaufnahmeelements mit den Merkmalen des Anspruchs 1 gelöst.

Die weitere Aufgabe, eine verbesserte Vorrichtung zu schaffen, mit der die Konzentration eines Liquids wie eines Kühlschmierstoffes zugleich mit weiteren Parametern wie dem Brechungsindex und der Temperatur und optional noch weiteren Parametern, die die Beschaffenheit des Fluids kennzeichnen, auf verbesserte Weise reproduzierbar und zuverlässig direkt vor Ort gemessen bzw. bestimmt werden kann, wird durch das Analysenvorrichtungs-Set mit den Merkmalen des Anspruchs 7 gelöst.

Die noch weitere Aufgabe, ein verbessertes und gleichzeitig vereinfachtes Messverfahren zur Analyse von mehr als drei Parametern eines Liquids wie eines Kühlmittelschmierstoffes vor Ort zu schaffen, wird durch das Verfahren mit den Merkmalen des unabhängigen Anspruchs 15 gelöst.

Weiterbildungen und bevorzugte Ausführungsformen der Vorrichtungen und des Verfahrens sind in den Unteransprüchen ausgeführt.

Eine erste Ausführungsform des erfindungsgemäßen Probeaufnahmeelements für eine Liquidprobe wie bspw. einen Kühlschmierstoff zur simultanen Analyse von drei oder mehr chemisch-physikalischen Parametern des Liquids, also quasi Kenndaten desselben, mittels einer Analysevorrichtung, in erster Linie um die Konzentration zumindest eines Inhaltsstoffes zu ermitteln, umfasst neben einem mit dem Liquid befüllbaren Probenaufnahmeraum zumindest drei benachbart zueinander angeordnete Messstellen, von denen eine eine photonische Messstelle (hierunter werden Messstellen für Absorption und Photolumineszenz zusammengefasst) und eine andere eine Brechungsindex-Messstelle ist.

Zudem weist das Probenaufnahmeelement zumindest eine weitere Messstelle auf, bei der es sich beispielsweise um eine pH-Messstelle, eine Leitfähigkeits-Messstelle oder eine Keimmessstelle handeln kann. Alle Messstellen sind über den Probenaufnahmeraum verteilt, d. h., dass bestimmte Bereiche des Probenaufnahmeraums jeweils eine Messstelle bilden, sodass die Messstellen, wenn Liquid in dem Probenaufnahmeraum des Probeaufnahmeelements aufgenommen ist, mit dem Liquid in fluidischem Kontakt stehen.

"Messstelle" bezeichnet somit einen vorbestimmten Bereich des Probenaufnahmeraums, der für die jeweils dort an dem Liquid vorgesehene Messung entsprechend ausgebildet ist: Wenn die vorgesehene Messung eine optoelektronische Messvorrichtung, etwa zur Erfassung von Photolumineszenz, vorsieht, mit der Licht auf den vorbestimmten Bereich des Probenaufnahmeraums gerichtet wird und von dem Liquid emittierte Lumineszenz erfasst wird, so weist die Messstelle des Probenaufnahmeelements entsprechend transparente Fenster beidseitig des Probenaufnahmeraums im entsprechenden Bereich auf, an denen (den Fenstern) das Liquid unmittelbar anliegt. In einem anderen Beispiel, wenn die durchzuführende Messung die Leitfähigkeit über den elektrischen Widerstand des Liquids bestimmen soll, erstrecken sich Elektroden bis in den Probenaufnahmeraum, wobei die Messstelle durch den Abstand ("Messstrecke") zwischen den Elektroden gebildet wird, der bei befülltem Probenaufnahmeraum mit dem Liquid in unmittelbarem Kontakt steht.

Für die benachbarte Anordnung mehrerer Messstellen ist das erfindungsgemäße Probeaufnahmeelement ein flächiges Element, das mit planparallel aufeinander angeordneten und zumindest abschnittsweise an ihren Rändern miteinander verbundenen Platten doppelwandig ausgeführt ist. Für die Brechungsindex-Messstelle weist dabei eine der Platten in dem dafür vorbestimmten Bereich, vorzugsweise auf der Innenseite der Platte, eine Prismenstruktur auf, mit der Lichtstrahlen, die durch die andere Platte eindringen und den Probenaufnahmeraum (und das darin befindliche Liquid) durchqueren, in vorbestimmter Weise gebrochen werden, wobei die Platten in dem vorbestimmten Bereich für die zur Brechungsindexmessung verwendeten Wellenlängen transparent sind. Dabei wird der Probenaufnahmeraum als ein Spalt flächig zwischen den Platten ausgebildet, wobei der Abstand zwischen den Doppelwänden gerade so groß ist, dass eine Liquidprobe an zumindest einer Stelle, an der die Platten am Rand nicht miteinander verbunden sind, mittels Kapillarwirkung in den Probenaufnahmeraum gezogen werden kann. Bei wässrigen Emulsionen mit einem Wassergehalt von zumindest 20 % kann dieser Abstand im Bereich von 0,5 bis 2 mm, vorzugsweise bei etwa 1 mm, liegen. Weist ein zu analysierendes Liquid einen davon abweichenden Wassergehalt oder eine abweichende Viskosität auf, so wird ein Probenaufnahmeelement mit einem entsprechend angepassten Abstand zwischen den Platten ausgeführt, um nach wie vor eine rein durch Kapillarkräfte bewirkte Befüllung des Probenaufnahmeraums zu erzielen. So ist es ausreichend, das Probenaufnahmeelement mit der Öffnung, die durch die nicht verbundenen Randstellen gebildet wird, in das Liquid, das untersucht werden soll, einzutauchen, bzw. die Liquidoberfläche zu kontaktieren - denn ein vollständiges Eintauchen ist nicht nötig - woraufhin die Liquidprobe durch Kapillarwirkung in den Probenaufnahmeraum hineinfließt. Gerade für Fluide wie Kühlschmiermittel ist dies ein sehr geeignetes Vorgehen. Vorteilhafterweise sind mit der rein passiven Befüllung durch Kapillarwirkung keinerlei Hilfsmittel erforderlich, um die Liquidprobe in den Probenaufnahmeraum des Probenaufnahmeelements zu bringen, wie dies im Stand der Technik der Fall ist, in dem entweder Pipetten zur Probennahme und Befüllung des Probenaufnahmeelements verwendet werden, das dann eine Rotationsbewegung ausführt, um das Liquid zu den Messstellen zu bringen, oder Probenaufnahmeelemente verwendet werden, die mit einer Pumpe verbunden werden müssen, um das Liquid in den Probenaufnahmeraum und zu den Messstellen zu ziehen.

So ergibt sich vorteilhaft, dass ein äußerst geringes Probenvolumen ausreichend ist, um eine Vielzahl von mindestens drei, eher vier oder mehr, unterschiedlichen chemisch-physikalischen Parametern zu messen.

Das als flächiges Element ausgeführte Probeaufnahmeelement kann besonders geeignet ein flacher, länglicher Probestreifen mit einer Gesamtdicke sein, die im Bereich von 2 bis 8 mm, bevorzugt im Bereich von 2,5 bis 6 mm und besonders bevorzugt im Bereich von 2,5 bis 4,5 mm liegt.

Dabei ist für die Gestaltung eines möglichst kleinen Probeaufnahmeelementes zu beachten, dass die Messstellen möglichst nahe beieinander liegen, quasi auf einem Fluidpfad, der von der Einlassöffnung, die spaltartig ausgebildet sein kann - es können auch mehrere Einlassöffnungen vorliegen - an den Messstellen entlang zu einer als Entlüftungskanal mit Luftaustrittsöffnung vorgesehenen Auslassstelle führt, an der die Platten ebenfalls nicht miteinander verbunden sind.

Um eine komfortable Befüllung zu ermöglichen, sind die Platten vorzugsweise zumindest entlang einer Seite des flächigen, insbesondere als länglicher Probestreifen ausgeführten Probenaufnahmeelements nicht miteinander verbunden, sodass ein Befüllspalt für das Liquid bereitgestellt wird. Bevorzugt kann es sich dabei um eine lange Seite des Probenstreifens handeln, da durch den längeren Befüllspalt eine deutlich kürzere Befüllzeit des Probenaufnahmeraums erreicht werden kann als durch einen Befüllspalt an einer kurzen Seite.

So kann ein schmaler, dünner Teststreifen aus zwei Plättchen, geschaffen werden, an dessen einer langen Seite an einer oder mehreren Stellen die Plättchen am Rand nicht miteinander verbunden, resp. verklebt sind, und in dessen Innerem dadurch, dass die Plättchen auch dort nicht verklebt sind, ein dünner Spalt wie ein Kanal gebildet wird, der das Liquid führt und entlang dem die Messstellen liegen. Für eine vollständige Befüllung des Probenaufnahmeraums kann sich der Entlüftungskanal durch eine kurze Seite erstrecken und nach außen münden, um Luft, die bei der Probenaufnahme durch Eindringen des Liquids aus dem Probenaufnahmeraum verdrängt wird, auszulassen.

Da das Probenaufnahmeelement Messstellen aufweist, an denen optische bzw. optoelektronische Analysenverfahren angewendet werden, ist das flächige Element vorteilhaft zumindest teilweise - also zumindest im Bereich der hierzu vorgesehenen Messstellen - aus lichtdurchlässigem Material wie Glas bzw. Quarzglas oder aus einem transparenten Kunststoff, wie beispielsweise Polymethylmethacrylat oder Polycarbonat beschaffen. Es kommen aber auch andere transparente Kunststoffe in Frage.

Zur Gestaltung der Messstelle für die Leitfähigkeitsmessung sind auf einem verlängerten Abschnitt einer der Platten, der über die andere Platte hinausragt, zumindest zwei Kontaktstreifen zur Spannungsbeaufschlagung angeordnet, die sich als Elektroden bis in den Probenaufnahmeraum erstrecken und dort durch eine Messstrecke, die die Leitfähigkeits-Messstelle bildet, voneinander beabstandet enden.

Vorteilhaft ist nach einer weiteren Ausführungsform an einem von dem Ende mit den Kontaktstreifen der Leitfähigkeits-Messstelle abgewandten Ende das flächige Element als ein Griffabschnitt zur Handhabung des Probenaufnahmeelements ausgebildet. Der von dem Probenaufnahmeraum ausgehende Entlüftungskanal kann sich durch diesen Griffabschnitt erstrecken und dort an einer Luftaustrittsöffnung austreten. Da in der Kombination mit dem als Handgerät ausgebildeten Analysengerät der Erfindung vorgesehen ist, dass der Griffabschnitt des Probenaufnahmeelements während des Messvorgangs teilweise aus dem Analysengerät ragt, kann auch eine andere Anordnung eines Entlüftungskanals vorgesehen sein, etwa, wenn dieser als Messstelle für eine Keimmessung mittels einer (Mikro-) Gas-Sensorik vorgesehen ist. Ein von dem Probenaufnahmeraum ausgehender Entlüftungskanal tritt dann an einer anderen Stelle aus, wo seine Luft- bzw. Gasaustrittsöffnung mit einer entsprechenden (Mikro-) Gas-Sensorik eines Analysengeräts kommunizieren kann.

Ferner kann der Griffabschnitt opak, bevorzugt schwarz, sein, um Streulichteinfall zu verhindern, wenn das Probenaufnahmeelement in das Analysengerät eingeführt ist. Es ist aber auch denkbar, unterschiedlich farbige Griffabschnitte für unterschiedliche Probenaufnahmeelemente vorzusehen. Ein Probenaufnahmeelement, das vollständig von einem Analysengerät aufgenommen wird, kann auch komplett transparent ausgeführt sein. Weiter kann ein Griffabschnitt eine strukturierte Oberfläche aufweisen, um die Handhabung durch eine bessere Griffigkeit zu erleichtern. Ferner können am Griffabschnitt - oder auch an anderen geeigneten Stellen des Probenaufnahmeelements Markierungen angebracht sein, um das richtige Einführen des Probenaufnahmeelements in ein Analysengerät zu unterstützen. Das richtige Einführen kann nach dem Schlüssel-Schloss-Prinzip auch durch spezielle Formgebung des Probenaufnahmeelements insbesondere an dem vom Griffabschnitt abgewandten Ende unterstützt werden.

Vorzugsweise handelt es sich bei der photonischen Messstelle um eine Lumineszenz-Messstelle und besonders bevorzugt um eine Fluoreszenz-Messstelle. Hierzu weist das Probenaufnahmeelement an beiden Platten in dem für die Messstelle vorgesehenen Bereich einen Fensterabschnitt auf, der für die entsprechenden Anregungs- und Emissionswellenlängen transparent ist. Die beiden Fensterabschnitte der Messstelle für die Lumineszenzmessung können kongruent sein.

Wenn das Probenaufnahmeelement eine pH-Messstelle aufweist, so kann diese ein indikatorfarbstoffhaltiges Substrat aufweisen, das an einem vorbestimmten zweiten Abschnitt zwischen den beiden Platten angeordnet ist, die entsprechend in einem Bereich, der diesen Abschnitt umgibt, für das zur optoelektronischen Erfassung der Farbänderung des Indikatorsubstrats erforderliche Licht transparent sind.

Zudem kann die Gruppe, aus der die zumindest eine weitere Messstelle ausgewählt wird, eine Nitrit-Messstelle umfassen, die vergleichbar der pH-Messstelle in Bezug auf eine optoelektronische Erfassung einer Farbänderung, jedoch mit einem nitritreaktiven Substrat ausgeführt sein kann. Hierfür kommt z. B. ein primäres, aromatisches Amin in Frage, das mit salpetriger Säure zu einem Diazoniumsalz reagiert, das wiederum in sauer Lösung mit Aminen eine farbige Azoverbindung bildet, die photometrisch nachweisbar und mit Kalibrierung quantifizierbar ist. Ein bekanntes Reagenz (Lunges Reagenz, photometrischer Nachweis der Azoverbindung bei 535 nm) besteht aus 1-Naphthylethylendiamin und Sulfanilsäure und ggf. Essigsäure. Die analytische Überwachung des Nitritgehalts in wässrigen Metallbearbeitungslösungen oder Emulsionen ist wichtig, da Nitrit als Reaktionspartner mit sekundären Aminen oder Alkanolaminen zu kanzerogenen Nitrosaminen reagieren kann. Nitrit kann unter anderem über das Ansetzwasser zur Emulsionsbildung oder über die Metallteile, die einen Härteprozess durchlaufen und noch mit Härtesalzen kontaminiert sind, in die Prozessmedien, d. h. die wässrigen Metallbearbeitungslösungen oder Emulsionen gelangen.

Zur Bildung der Brechungsindex-Messstelle ist erfindungsgemäß an einem vorbestimmten dritten Abschnitt einer der beiden Platten die Prismenstruktur oder eine Fresnelsche Linsenstruktur, die eine spezielle Prismenstruktur darstellt, vorgesehen. Auch hier sind die Platten an dem Abschnitt für die zur Brechungsindexmessung verwendeten Wellenlängen transparent. Die Prismenstruktur stellt in Bezug auf die Plattenebene abgewinkelte Flächenabschnitte bereit, an denen auftreffende Lichtstrahlen entsprechend umgelenkt werden. Eine Prismenstruktur besteht aus wenigstens einer, vorzugsweise mehreren, benachbart angeordneten, im Profil dreieckigen Strukturen. Eine Fresnelsche Linsenstruktur weist eine Reihe ringförmiger Stufen auf.

Der Abschnitt mit der Prismenstruktur bzw. der Fresnelschen Linsenstruktur sowie der Abschnitt mit dem indikatorfarbstoffhaltigen Substrat bilden dabei konstituierende Komponenten für die optischen, elektronischen und optoelektronischen Analysevorrichtungen, mit denen das Probeaufnahmeelement während einer Messung bzw. eines Analysenvorganges korrespondiert.

Generell ist dabei vorgesehen, dass das erfindungsgemäße Probeaufnahmeelement als Einmal-Messstreifen ausgebildet ist.

Ein ebenfalls erfindungsgemäßes Analysevorrichtungs-Set für die simultane Analyse von drei oder mehr chemisch-physikalischen Parametern bzw. Kenndaten von Liquiden umfasst ein als Handgerät ausgebildetes Analysegerät mit einem Gehäuse und mit einer Anzeige sowie zumindest ein erfindungsgemäßes Probenaufnahmeelement für die Liquidprobe. Handgerät bedeutet dabei, dass das Gerät klein und handlich ist und ohne weiteres von einer Person zu den Anlagen, die das zu analysierende Liquid verwenden, getragen und händisch bedient werden kann. Für die an dem Probenaufnahmeelement durchzuführenden Messungen weist das Analysengerät eine optoelektronische Analysenvorrichtung auf, die zumindest drei benachbart zueinander angeordnete Messvorrichtungen aufweist, deren Anordnung mit der Anordnung der Messstellen auf dem Probenaufnahmeelement korrespondiert. Zudem weist das Analysengerät eine Datenverarbeitungseinheit auf, die mit der Analysenvorrichtung und der Anzeigevorrichtung kommunikativ verbunden ist.

In dem Gehäuse des erfindungsgemäßen Analysegeräts befindet sich eine Einsteckvorrichtung zur Aufnahme des Probenaufnahmeelements, die lösbar in dem Gehäuse angeordnet ist und eine Einstecköffnung aufweist. Diese mündet in eine Ausnehmung, die zur Aufnahme des Probeaufnahmeelements korrespondierend dazu ausgebildet ist. Weiter weist die Einsteckvorrichtung korrespondierend zu den Anordnungen der Messvorrichtungen und der Messstellen jeweils in Abhängigkeit der Art der jeweiligen Messstelle eine optische, elektronische oder optoelektronische Kommunikationseinrichtung auf, die eine entsprechende Signalübertragung (hierunter wird auch Lichttransmission verstanden) zwischen den Messstellen eines in der Einsteckvorrichtung aufgenommenen Probenaufnahmeelements und den Messvorrichtungen gestattet.

Hierzu ist die Einsteckvorrichtung zumindest teilweise aus transparentem Material gefertigt. Das heißt, sie ist zumindest an den Stellen durchsichtig, an denen dies für optische Messungen erforderlich ist. Generell kann die Einsteckvorrichtung aus opakem Material, bevorzugt aus Kunststoff, besonders bevorzugt aus schwarzem Kunststoff gefertigt sein und ist dann auch für Störlicht unempfänglich.

Die Einsteckvorrichtung kann mit einem Flanschabschnitt, der die Einstecköffnung aufweist, und einem Hüllabschnitt ausgebildet sein, der in dem Gehäuse lösbar angeordnet ist, die Ausnehmung begrenzt und die optischen, elektronischen oder optoelektronischen Kommunikationseinrichtungen aufweist. Zwar sind diese vorzugsweise fensterartig durch Abschnitte aus transparentem Material ausgeführt - denn nur so wird eine Verschmutzung des Innenraums des Analysengeräts verhindert - es ist aber auch denkbar, dass diese Kommunikationseinrichtungen lediglich durch Öffnungen in dem Hüllabschnitt gebildet werden. Durch die optischen, elektronischen oder optoelektronischen Kommunikationseinrichtungen können die Komponenten des Analysengerätes und des Probeaufnahmeelements zusammenwirken, um die Analyse der zu bestimmenden chemisch-physikalischen Parameter zu ermöglichen. In Bezug auf die Brechungsindexmessung stehen beispielsweise der Abschnitt mit der Prismenstruktur und eine entsprechende Lichtquelle über ein Fenster in der Einsteckvorrichtung derart in Kommunikation, dass Licht durch das Fenster und durch das im Probenaufnahmeraum aufgenommene Liquid auf den Abschnitt mit der Prismenstruktur trifft und dort gebrochen wird. Ein weiteres Fenster auf der anderen Seite der Einsteckvorrichtung ermöglicht dann die Kommunikation mit einem Sensor des Analysengeräts zur Bestimmung des Brechungswinkels.

Zwei der Messvorrichtungen des Analysengeräts sind eine photonische Messvorrichtung, bevorzugt eine Lumineszenzmessvorrichtung, besonders bevorzugt eine Fluoreszenzmessvorrichtung, die bei Verwendung von Fluoreszenzmarkern im Liquid zur Konzentrationsmessung einer oder auch mehrerer, auch verschiedener Komponenten des Liquids dient, und eine Brechungsindex-Messvorrichtung. Die Lumineszenzmessvorrichtung weist eine Anregungslichtquelle mit einer zur Anregung des Fluoreszenzmarkers geeigneten Wellenlänge auf, und eine zur Messung der emittierten Fluoreszenz geeignete Sensorik. Die Brechungsindex-Messvorrichtung des Analysengeräts weist bis auf die Prismenstruktur, die, wie oben ausgeführt, Teil des Probenaufnahmeelements ist, alle übrigen erforderlichen Komponenten des Refraktometers wie Lichtquelle und Sensorik auf.

Analog zu der zumindest einen weiteren Messstelle des Probenaufnahmeelements weist das Analysengerät zumindest eine weitere Messvorrichtung auf, die korrespondierend zu den Messstellen des Probenaufnahmeelements aus der Gruppe ausgewählt ist. Dabei kann es sich beispielsweise um eine pH-Messvorrichtung handeln, die bevorzugt als pH-Optode ausgeführt sein kann, wobei der optische Effekt der Farbänderung des Indikatorsubstrats bei Kontakt mit dem zu untersuchenden Liquid genutzt wird. Wird als Indikatorsubstrat ein Indikatorpapier eingesetzt, wird eine Messvorrichtung genutzt, bei der die von dem Indikatorpapier reflektierte Lichtfarbe detektiert wird.

Vorzugsweise kann ein Universalindikator mit einer Mischung mehrerer Indikatorsubstanzen mit verschiedenen Farben und verschiedenen Umschlagsbereichen eingesetzt werden, die so abgestimmt sind, dass pH-Werte in einem weitem pH-Bereich durch verschiedene Farbumschläge erkennbar gemacht werden können.

Wenn das Probenaufnahmeelement zum Nachweis von Nitrit bzw. zur Messung des Nitritgehalts eine Nitrit-Messstelle aufweist, so ist ein entsprechend mit einer Nitrit-Messvorrichtung ausgestattetes Analysengerät zur Analyse zu verwenden.

So ist sowohl für die Lumineszenzmessvorrichtung und die Brechungsindex-Messvorrichtung, als auch für die pH-Messvorrichtung und die Nitrit-Messvorrichtung jeweils eine Lichtquelleneinheit, mit der nicht nur die Lichtquelle, sondern auch gegebenenfalls erforderliche optische Bauelemente wie Filter, Linsen etc. umfasst sein sollen, sowie eine Detektionseinheit (ebenfalls umfassend optische Bauelemente wie Filter, Linsen etc. und den eigentlichen Detektor) vorgesehen. Die verschiedenen Messvorrichtungen können unterschiedliche Lichtquellen und Detektoren aufweisen, die je nach Messprinzip ausgewählt werden - diese Auswahl ist dem Fachmann bekannt. In dem Analysengerät können die Lichtquelleneinheiten der verschiedenen Messvorrichtungen auf der einen Seite des Probenaufnahmeelements bzw. der Einsteckvorrichtung, und die Detektoreinheiten auf der anderen Seite angeordnet sein. Die Strahlengänge zwischen den Lichtquellen und den Detektoren verlaufen durch entsprechende Anordnung bzw. den Einsatz entsprechender optischer Bauelemente so, dass die Lichtstrahlen das Probenaufnahmeelement an den jeweiligen Messstellen durch dringen (Lumineszenz und Brechung) oder dort reflektiert werden (pH).

Als zur pH-Messvorrichtung alternative oder zusätzliche Messvorrichtung kann ein Analysengerät auch eine Leitfähigkeits-Messvorrichtung aufweisen, bei der es sich eigentlich um eine Widerstandsmessvorrichtung handelt, und bei der aus dem gemessenen Widerstand die Leitfähigkeit des Liquids bestimmt wird. Auch hier weist das Probenaufnahmeelement mit den Kontaktstreifen einen Teil der Messvorrichtung auf. Die Leitfähigkeits-Messvorrichtung des Analysengeräts weist einen Frequenzgenerator mit Kontaktelementen auf, die nach Anordnung des Probenaufnahmeelements in dem Analysengerät direkt oder indirekt über Kontaktbrückenelemente mit den zumindest zwei Kontaktstreifen des Probenaufnahmeelements in elektrischem Kontakt stehen.

Zur Erfassung der Keimbelastung des Liquids kann das Analysengerät eine entsprechende Messvorrichtung aufweisen, bei der es sich um eine sogenannte "elektronische Nase" handeln kann, die aus zumindest einem mikroelektronischen Gassensor, üblicherweise aus einer Mehrzahl von Gassensoren, gebildet wird, da Keime flüchtige organische Verbindungen erzeugen, die aus dem Liquid in die Dampfphase übergehen und mit den Gassensoren detektiert werden können, wenn diese Dampfphase mit den Sensoren in Verbindung gebracht wird. Dazu kann der Entlüftungskanal des Probenaufnahmeelements über eine Verbindungsleitung des Analysengeräts mit der elektronischen Nase in Verbindung gebracht werden. Die Verbindungsleitung kann auch zu dem Befüllspalt führen - denkbar als Keimmessstelle könnte auch ein entsprechend gaspermeables Fenster in zumindest einer der Platten sein, durch das die flüchtigen Verbindungen über die Verbindungsleitung zu den Gassensoren gelangen. Um einen gerichteten Zustrom der flüchtigen Verbindungen zu den Gassensoren zu erhalten, ist der Einsatz eines Mikrogebläses denkbar; eine gerichtete Strömungsführung kann auch durch ein spezielles Design des Entlüftungskanals und der Verbindungsleitung hinsichtlich Querschnittsgestaltung unterstützt werden.

Da insbesondere der Brechungsindex temperaturabhängig ist, weist das Analysengerät eine Temperaturmessvorrichtung auf, die mit der Datenverarbeitungseinheit verbunden ist, sodass der Einfluss der Temperatur bei der Messung des Brechungsindex' kompensiert werden kann. Der eingesetzte Temperatursensor kann beispielsweise ein Widerstandsthermometer sein, das aufgrund seiner geringen Ausmaße gut im Gehäuse des als Handgerät ausgeführten Analysengeräts untergebracht werden kann.

Eine Verschmutzung der sensiblen Messtechnik im Inneren des Analysengeräts wird durch die bereits beschriebene Einsteckvorrichtung verhindert, die das eingesteckte Probenaufnahmeelement von dem Innenraum des Analysengeräts trennt, dessen Gehäuse entsprechend fluid- und staubdicht ausgeführt ist. Der Flanschabschnitt der Einsteckvorrichtung einer bevorzugten Ausführungsform liegt in einer Analyseanordnung, in der die Einsteckvorrichtung in das Gehäuse eingesteckt ist, an einem Rand des Gehäuses außenseitig an und fasst eine Abdeckplatte ein, in die die Einstecköffnung eingebracht ist. Diese Einstecköffnung kann durch eine Dichtlippe bzw. ein Dichtlippenpaar abgedichtet werden, sodass an der Außenseite des Probeaufnahmeelements gegebenenfalls vorhandenes Liquid beim Einführen abgestreift wird und somit nicht in das Analysengerät eindringt. Die Dichtlippe(n) werden von der Abdeckplatte in dem Flanschabschnitt gehalten, wobei die Abdeckplatte lösbar in dem Flanschabschnitt befestigt bspw. verschraubt ist. Dabei ist es auch möglich, dass die Schrauben nicht nur zur Befestigung der Abdeckplatte in dem Flanschabschnitt ausgelegt sind, sondern den Flanschabschnitt durchdringen und somit gleichzeitig für die lösbare Befestigung der Einsteckvorrichtung an dem Gehäuse des Analysengeräts sorgen. Es sind aber auch andere Befestigungsvarianten sowohl der Abdeckplatte in dem Flanschabschnitt als auch der Einsteckvorrichtung im Analysengerät denkbar; so kommen auch Steck-, Klemm- oder Rastsysteme in Frage.

Für die Leitfähigkeitsmessung kann alternativ zur oben beschriebenen direkten Kontaktierung der Kontaktstreifen des Probenaufnahmeelements mit den Kontaktelementen des Frequenzgenerators vorgesehen sein, dass die Einsteckvorrichtung Kontaktbrücken aufweist, die den Kontakt der Kontaktelemente des Analysengeräts mit den zumindest zwei Kontaktstreifen des Probenaufnahmeelements herstellen, wenn dieses in der Analysenanordnung in der Einsteckvorrichtung angeordnet ist.

Die Kontaktbrücken und/oder die Kontaktelemente des Analysengeräts können als Kontaktfedern bzw. Federkontaktleiste ausgebildet sein, um die sichere Kontaktierung mit den Kontaktstreifen des eingeschobenen Probenaufnahmeelements zu leisten.

Der Frequenzgenerator und alle übrigen elektrischen Verbraucher des Analysengeräts, wie die optoelektronische Analysenvorrichtung, die Datenverarbeitungseinheit und die Anzeigevorrichtung sowie der Thermosensor etc., sind mit einer Energiequelle verbunden, die ebenfalls im Gehäuse des Analysengeräts untergebracht ist. Die Energiequelle kann vorzugsweise ein Akkumulator sein, der über eine Schnittstelle im Gehäuse wieder aufgeladen werden kann. Gegebenenfalls kann das Analysengerät an der Außenseite auch eine oder mehrere Solarzellen zum Wiederaufladen des Akkumulators aufweisen.

Die Anzeigenvorrichtung kann als berührungssensitive Anzeigevorrichtung (nachfolgend auch Touchscreen-Display) ausgebildet sein, und damit gleichzeitig eine Bedienschnittstelle darstellen, um Benutzereingaben über die Kommunikationsleitung an die Datenverarbeitungseinheit zu übermitteln. Diese kann eine externe Kommunikationsschnittstelle aufweisen oder damit verbunden sein, die eine Steckerschnittstelle wie z. B. USB- oder Mikro-USB-Schnittstelle oder eine Funkschnittstelle, insbesondere Nahbereichsfunkschnittstelle z. B. nach dem Bluetooth®-Standard etc. sein kann.

Ein weiterer erfindungsgemäßer Gegenstand ist ein Verfahren zur simultanen Analyse zumindest dreier unterschiedlicher chemisch-physikalischer Parameter eines Liquids in situ unter Verwendung eines erfindungsgemäßen Analysenvorrichtungs-Sets. Das Verfahren umfasst die Schritte:
- Eintauchen des Probenaufnahmeelements in das Liquid oder Kontaktieren der Liquidoberfläche mit einer Öffnung des Probenaufnahmeelements, die durch die nicht verbundenen Randstellen gebildet wird, und Füllen des Probenaufnahmeraums des Probenaufnahmeelements mit einer Probe des zu untersuchenden Liquids durch die Kapillarwirkung zwischen den Doppelwänden des Probenaufnahmeelements, wozu die Befüllöffnung für eine vorbestimmte Dauer, die von den Abmessungen des Probenaufnahmeraums und der Befüllöffnung abhängig ist, in das Liquid getaucht wird,
- vollständig Einstecken des Probenaufnahmeelements in das Analysengerät,
- Starten und Durchführen von zumindest drei oder mehr Messvorgängen gleichzeitig durch die Mess-Vorrichtungen an den Messstellen,
- nach Beendigung der Messvorgänge Anzeigen der Messergebnisse auf der Anzeigevorrichtung.

Vorteilhaft kann dabei in einer Weiterbildung des Verfahrens aus verschiedenen untersuchbaren Liquiden, die in einer Datenbank hinterlegt sind, die in der Datenverarbeitungseinheit oder auf einem damit verbundenen Speichermedium gespeichert sind, und die in einem Auswahlmenü angeboten werden, ein zu untersuchendes Liquid durch eine Benutzereingabe auf der Anzeigenvorrichtung, die geeigneter Weise als Touchscreen-Display ausgeführt sein kann, ausgewählt werden.

Generell ist es aber möglich, das Analysengerät für einen bestimmten Liquid-Typus auszulegen, um ein besonders einfaches Gerät für einen ganz bestimmten Anwendungsfall zu schaffen, sodass keine Liquidauswahl erfolgen muss.

Ebenfalls optional kann in Weiterbildungen des Verfahrens vorgesehen sein, nach Beendigung der Messvorgänge auf der Anzeigenvorrichtung eine Aufforderung zum Entfernen des Probenaufnahmeelements aus dem Analysengerät anzuzeigen. Die Entnahme wird über die Software erfasst, wenn der Messvorgang abgeschlossen ist. Beim Hineinstecken des Probenaufnahmeelements hingegen wird die Endlage opto-elektronisch erfasst und dann die Auswertung und Datenerfassung gestartet, was automatisch oder durch Benutzereingabe erfolgen kann. Schließlich ist es erfindungsgemäß auch möglich, nach erfasster Entnahme des Probenaufnahmeelements aus dem Analysengerät die Messergebnisse auf der Anzeigevorrichtung anzuzeigen und auch zu Speichern und/oder an weitere Geräte zu übertragen.

Das Speichern kann in einem internen Speicher der Datenverarbeitungseinheit oder einem damit verbundenen Wechselspeichermedium, wie etwa einer SD-Karte oder einem USB-Stick, erfolgen. Die Übertragung der Messergebnisse kann vorzugsweise über die Funkschnittstelle an einen voreingestellten Empfänger, aber auch verdrahtet über ein entsprechendes USB-Kabel erfolgen.

Ausführungsformen des Verfahrens beziehen sich auf das Kalibrieren des Analysengeräts für die untersuchbaren Liquide, die in der Datenbank hinterlegt sind, und/oder das Einlernen neuer Liquide mit dem Analysengerät und Hinzufügen der eingelernten Liquide in die Datenbank. Beides, Kalibrieren und Einlernen, erfolgt jeweils durch Auswahl und Bestätigung entsprechender Felder, die im Auswahlmenü angezeigt werden, wobei zum Kalibrieren Kalibrierlösungen mit bekannten chemisch-physikalischen Parametern bereitgestellt werden, um die Messvorrichtungen zu kalibrieren. Zum Einlernen neuer Liquide werden diese als zu untersuchende Liquide mit bekannten chemisch-physikalischen Parametern bereitgestellt.

Auch bei dem Verfahren ist das Liquid ein Liquid, das zumindest eine mittels Lumineszenzanalyse nachweisbare Markersubstanz aufweist, wobei eine der Messstellen eine Lumineszenz-Messstelle ist.

Insbesondere kann das Verfahren unter Verwendung eines erfindungsgemäßen Analysenvorrichtungs-Sets zur Analyse einer Metallbearbeitungsflüssigkeit, insbesondere eines Kühlschmierstoff, dabei vor allem einer Kühlschmierstoffemulsion als Liquid angewendet werden, wobei dem Liquid zumindest eine erste mittels Lumineszenzanalyse nachweisbare Markersubstanz in einer vorbestimmten Konzentration zugesetzt ist, sodass über die Lumineszenzmessung Rückschlüsse auf die Konzentration eines Liquidinhaltsstoffes, insbesondere auf die Konzentration des Kühlschmierstoffs, in der Emulsion möglich sind.

Zur Bestimmung der Kühlschmierstoffkonzentration in einer Emulsion mittels Lumineszenzanalyse wird die Markersubstanz der Kühlschmierstoffemulsion in einer vorbestimmten Konzentration zugegeben. Die molare Konzentration des Markers oder der Markerzusammensetzung, die auch aus mehreren Markern zusammengesetzt sein kann, liegt bei 10⁻⁵ bis 10⁻⁶ Mol/Liter im Kühlschmierstoffkonzentrat bzw bei 10⁻⁷ bis 10⁻⁸ Mol/Liter in der Anwendungskonzentration, also in der Emulsion des Kühlschmierstoffes. Diese Dosierung bezieht sich u. a. auf Farbstoffe der Basischemie Perylene. Der dem Liquid zur Konzentrationsmessung zugegebene Lumineszenzmarker kann ein mit dem bloßen Auge nicht sichtbarer oder auch ein sichtbarer Farbstoff sein.

Vorzugsweise kann ein Marker verwendet werden, der aus zumindest zwei Farbstoffmolekülen aus der Reihe der Rylenfarbstoffe, z. B. Perylen und Quaterrylen, oder einer Kombination von Rhodamincarbonylderivaten und Acridinderivaten aufgebaut ist, sodass zumindest zwei Messbereiche im langwelligen Bereich abgedeckt werden können. Bei simultaner Messung in zwei Messbereichen können Messfehler minimiert werden.

Handelt es sich bei dem Liquid um eine Kühlschmierstoffemulsion für spezielle Fertigungszwecke, dann kann zwecks Leistungserhöhung ein Booster beigegeben werden. Dies geschieht meistens mit einem Anteil von unter 5 Gewichtsprozenten in Bezug auf das Gesamtgewicht der Kühlschmierstoffemulsion. Bei der Fertigung von Kleinserien von Bauteilen mit Werkzeugmaschinen, die eigentlich nicht für Kleinserien gedacht sind, müssen zum Qualitätserhalt der Kleinserien derartige Booster zur Leistungsverbesserung des Kühlschmierstoffes eingesetzt werden, um zu vermeiden, einen speziellen Kühlschmierstoff für diese Anforderungen entwickeln zu müssen, was unwirtschaftlich wäre. In solchen Fällen ist es von besonderem Vorteil, anhand eines weiteren zugesetzten Markers, der den Booster kennzeichnet, die Konzentration des Boosters in situ ohne weiteres mit dem erfindungsgemäßen Probeaufnahmeelement und dem im Set zugehörigen Analysengerät bestimmen zu können. Bislang konnte dies nur im Labor durch Infrarotspektroskopie zur Detektion der Esterbande (wenn der Booster eine Esterverbindung enthält) und/oder durch Röntgenfluoreszenzanalyse zur Detektion von Schwefel-/PhosphorVerbindungen des Boosters festgestellt werden.

So bezieht sich das erfindungsgemäße Verfahren auch darauf, dass das Liquid ein Boosteradditiv aufweist und dem Liquid zumindest eine zweite mittels Lumineszenzanalyse nachweisbare Markersubstanz in einer vorbestimmten Konzentration zugesetzt wird, die sich von der ersten Markersubstanz hinsichtlich ihrer Lumineszenzeigenschaften unterscheidet, sodass bei der Lumineszenzanalyse über die Lumineszenz des ersten Markers die Konzentration eines ersten Inhaltsstoffes, z. B. des Kühlschmierstoffs in der Emulsion, und über die Lumineszenz des zweiten Markers auf die Konzentration des Boosteradditivs geschlossen werden kann.

Die Markerauswahl richtet sich geeigneter Weise nach Gesichtspunkten der Balance zwischen Oleophilie und Hydrophilie. Der Marker, wenn nur der Booster markiert wird, verbleibt in der Anwendung auch nur im Booster und diffundiert nicht in die Basisemulsion. Eine Erklärung dafür könnte das Nebeneinandervorliegen von unterschiedlichen Mycellstrukturen sein. So lassen sich mittels Partikelmessung bspw. mittels eines Coulter-Zählers feststellen, dass ein solches sogennantes "Two Pack System" aus Booster und Marker zu zwei Peaks führt, was auf das Vorliegen unterschiedlicher Mycellstrukturen schließen lässt. Diese Theorie könnte auch dadurch gestützt werden, dass ein solches Two Pack System in der Anwendung - im Vergleich zu einem System bei dem die Leistungsträger in ein Standardkonzentrat eingebaut wurden - eine höhere Leistung generiert. Dabei müssen zum Vergleich auch gleiche Konzentrationen betrachtet werden.

Letztlich wurden auch Fluoreszenzmessungen an den markierten Emulsionssystemen durchgeführt.

Das erfindungsgemäße Analysenvorrichtungs-Set aus Probeaufnahmeelement und Analysengerät ist daher auch sehr gut geeignet für die Untersuchung von Liquiden wie Metallbearbeitungsflüssigkeiten, Kühlschmierstoffen, Kühlschmierstoffemulsionen, die auch einen Booster enthalten können.

Unter Metallbearbeitungsflüssigkeiten werden im vorliegenden Fall sämtliche Flüssigkeiten verstanden, die zur Schmierung und/oder Kühlung sowie gegebenenfalls zur Spülung bei Metallbearbeitungsprozessen wie Umformen oder zerspanenden Prozessen wie Schneiden, Schleifen, Läppen, Isolieren/Erodieren eingesetzt werden. Häufig werden dabei Kühlschmierstoffe eingesetzt, die beide Funktionen der Kühlung und Schmierung und gegebenenfalls auch der Spülung verbinden. Auch können Kühlschmierstoffe bei der Minimalmengenschmierung eingesetzt werden. Kühlschmierstoffemulsionen wiederum beziehen sich auf entsprechend wassergemischte Zusammensetzungen. Zwar ist die Erfindung besonders vorteilhaft zur Analyse von derartigen Metallbearbeitungsflüssigkeiten bzw. Kühlschmierstoffen und dabei insbesondere von wässrigen Kühlschmierstoffemulsionen geeignet, jedoch in keiner Weise darauf beschränkt. So kann ein erfindungsgemäßes Probenaufnahmeelement, ein erfindungsgemäßes Analysenvorrichtungs-Set und ein erfindungsgemäßes Verfahren auch generell zur Analyse von jeglichen wasserhaltigen Fluiden eingesetzt werden, also beispielsweise auch von Getriebe- oder Hydraulikfluiden oder wässrigen Reinigungslösungen oder -emulsionen.

Das untersuchte Liquid ist bevorzugt ein wasserhaltiges Liquid mit einem Wassergehalt von 1 bis 99,9 %, besonders bevorzugt ist es ein wasserbasiertes Liquid mit einem Wassergehalt von 1 bis 15 %.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich schematische Darstellungen von beispielhaften Ausführungsform der Erfindung.

Dabei zeigen:
- **Fig. 1**: eine Draufsicht auf ein erfindungsgemäßes Probenaufnahmeelement,
- **Fig. 2**: eine perspektivische Draufsicht auf eine Einsteckvorrichtung eines erfindungsgemäßen Analysengeräts,
- **Fig. 2a**: eine schematische Seitenschnittansicht gemäß AA in Fig. 2,
- **Fig. 3**: eine Seitenansicht der Einsteckvorrichtung,
- **Fig. 4**: eine Seitenansicht des Analysengeräts mit Einsteckvorrichtung und eingestecktem Probenaufnahmeelement,
- **Fig. 5**: eine schematische Draufsicht auf eine Halbschale des Analysengeräts mit Einsteckvorrichtung und einer optoelektronischen Analysenvorrichtung,
- **Fig. 6**: eine schematische Darstellung einer optischen pH-Messvorrichtung der optoelektronischen Analysenvorrichtung,
- **Fig. 7**: eine schematische Darstellung eines Refraktometers der optoelektronischen Analysenvorrichtung,
- **Fig. 8**: eine schematische Darstellung eines Luminometers der optoelektronischen Analysenvorrichtung,
- **Fig. 9**: eine schematische perspektivische Ansicht auf ein aufgeklapptes Analysengerät mit Einsteckvorrichtung,
- **Fig. 10**: eine Seitenansicht des Analysengeräts mit Einsteckvorrichtung und eingestecktem Probenaufnahmeelement einer alternativen Ausführungsform des Analysenvorrichtungs-Sets,
- **Fig. 11**: eine Draufsicht auf ein erfindungsgemäßes Probenaufnahmeelement mit zusätzlicher Nitritmessstelle,
- **Fig. 12**: eine schematische Draufsicht auf eine optoelektronischen Analysenvorrichtung des Analysengeräts mit Einsteckvorrichtung für das Probenaufnahmeelement aus Fig. 11.

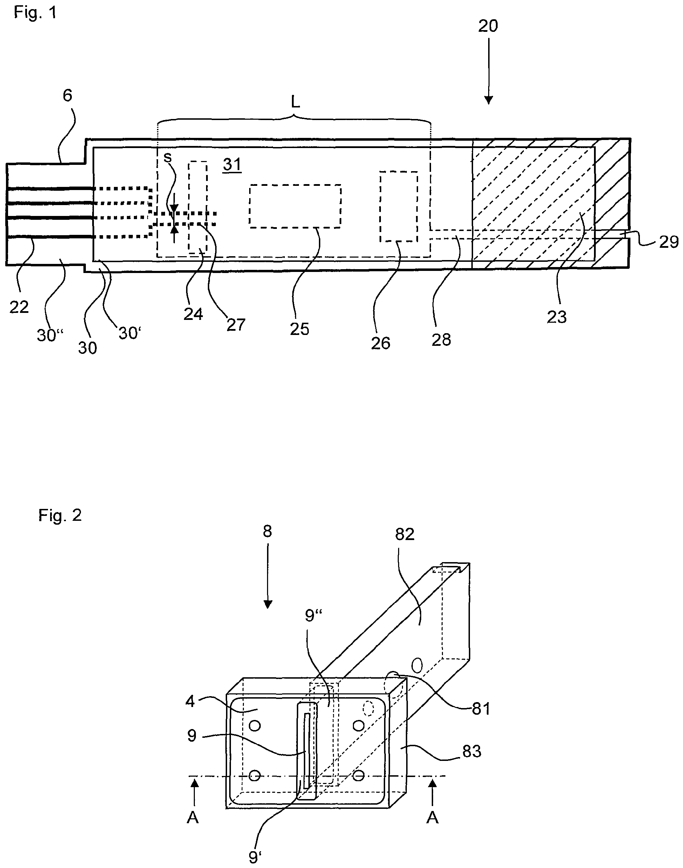

Das erfindungsgemäße Analysenvorrichtungs-Set bezieht sich auf ein als Handgerät ausgebildetes Analysengerät zur simultanen Bestimmung verschiedener Kenndaten einer Metallbearbeitungsflüssigkeit, insbesondere eines Kühlschmierstoffs, mobil vor Ort im Fertigungsbereich bzw. direkt an der Werkzeugmaschine unter Verwendung eines speziellen Probenaufnahmeelements. **Fig. 1** zeigt ein beispielhaftes Probenaufnahmeelement 20, das als Einmalteststreifen konzipiert ist.

Das Probenaufnahmeelement 20 ist hier ein näherungsweise rechteckiges Flächenelement, das einen als Spalt flächig ausgedehnten Probenaufnahmeraum 31 zwischen zwei Platten 30,30' aufweist, wozu die Abdeckplatte 30' bis auf eine zur Befüllung vorgesehene Öffnung der Länge L an ihren Rändern mit der Basisplatte 30 verbunden ist, die verschiedene Funktionsabschnitte und -elemente aufweist. Die Befüllöffnung kann, wie dargestellt, eine sich längs einer Längskante erstreckende durchgehende Spaltöffnung sein; je nach Abmessungen des Probenaufnahmeelements 20 können aber auch mehrere Befüllöffnungen vorgesehen sein, durch die der Probenaufnahmeraum 31 mittels Kapillareffekt gefüllt wird. So ist auch der Abstand zwischen den Platten 30,30' gerade so groß gewählt, dass die Liquidprobe infolge der Kapillarwirkung durch die Befüllöffnung vollständig und gleichmäßig in den Probenaufnahmeraum 31 gezogen wird. Somit ist die Breite des Spalts auch von den Dimensionen des Probenaufnahmeraums 31 abhängig, wird aber im Bereich von 0,1 bis 2 mm, bevorzugt bei 0,5 bis 1,5 mm, beispielsweise in etwa 1 mm, liegen um einen Probenaufnahmeraum 31 zu bilden. Eine geeignete Dimension für ein Probenaufnahmeelement 20 wurde bspw. mit 12 mal 28 mm getestet.

Das so in **Fig. 1** als Teststreifen ausgeführte Probenaufnahmeelement 20 kann somit eine Dicke, die im Bereich von 2 bis 8 mm, bevorzugt im Bereich von 2,5 bis 6 mm und besonders bevorzugt im Bereich von 2,5 bis 4,5 mm liegt, aufweisen. Ferner hängt die Größe und Form des Probenaufnahmeraums 31 und damit des Probenaufnahmeelements 20 auch von der Art, Anzahl und Flächenbedarf der Messstellen 24,25,26,27 ab, die alle innerhalb der Fläche des Probenaufnahmeraums 31 benachbart, aber vorwiegend auch voneinander beabstandet liegen müssen.

Die Befüllung des Probenaufnahmeraums 31 wird durch einen Entlüftungskanal 28 unterstützt, der sich zwischen den Platten 30,30' zu einer Luftaustrittsöffnung 29 erstreckt - d. h., dass die Platten auch im Bereich des Entlüftungskanals 28 nicht miteinander verbunden sind. Im hier gezeigten Beispiel verläuft der Entlüftungskanal 28 von einer zum Befüllspalt benachbarten Seite des Probenaufnahmeraums 31 durch einen Griffabschnitt 23. Es ist aber auch denkbar, Form, Anzahl und Anordnung der Entlüftungskanäle zu variieren.

Der Griffabschnitt 23 kann zum besseren Handhaben geriffelt sein oder andere Strukturen aufweisen.

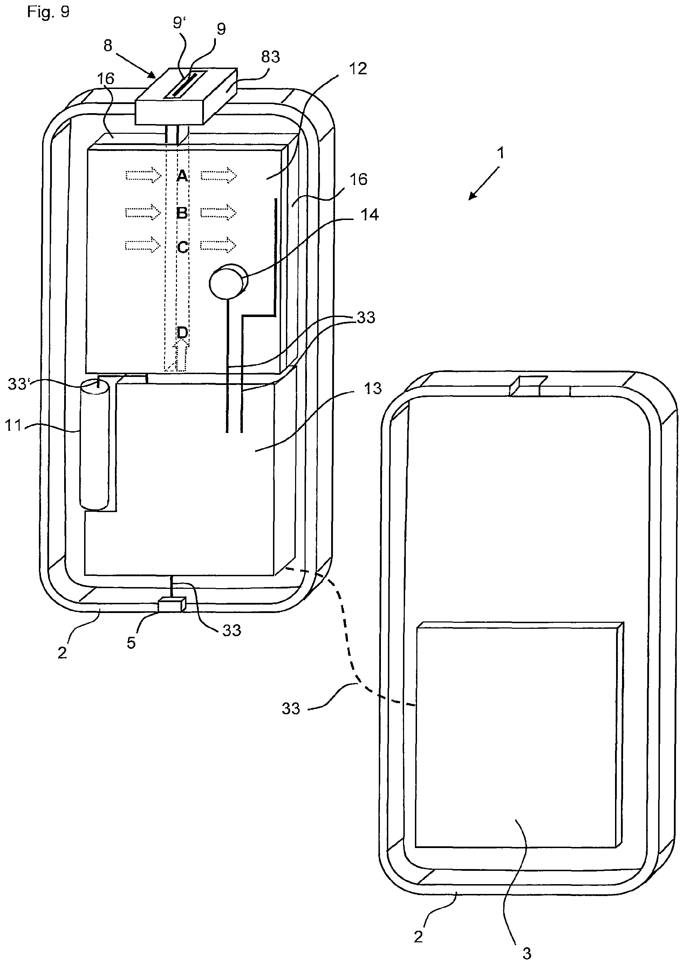

Über den Probenaufnahmeraum 31 verteilt weist das Probenaufnahmeelement 20 der **Fig. 1** drei benachbart zueinander angeordnete optische Messstellen 24,25,26 und eine Leitfähigkeitsmessstelle 27 auf, die sich im vorliegenden Beispiel mit zwei ihrer Kontaktstreifen 22 bis in den Bereich einer der optischen Messstellen 24 erstrecken. Mit diesem Probenaufnahmeelement 20 können daher mit dem entsprechenden Analysengerät 1 (vgl. **Fig. 9**) gleichzeitig drei optische Messungen A, B, C und eine Leitfähigkeitsmessung D durchgeführt werden, nachdem das mit Liquid befüllte Probenaufnahmeelement 20 durch die Einstecköffnung 9 in das Analysengerät 1 eingeführt wurde.

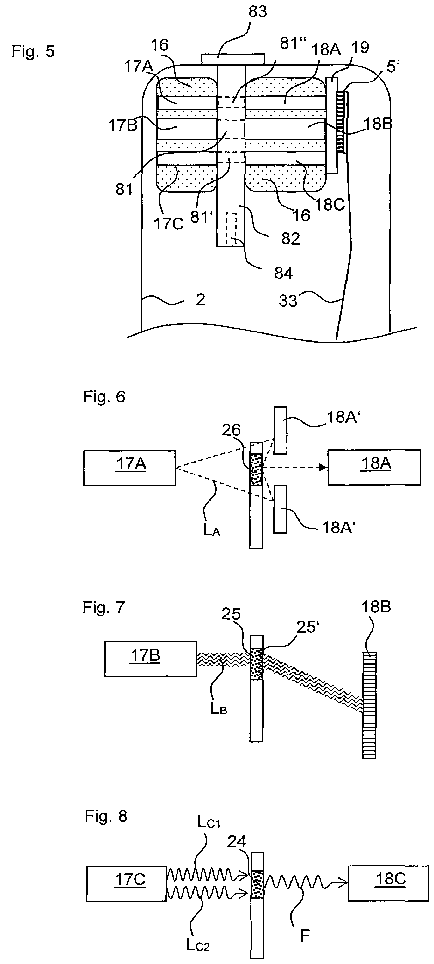

Die erste optische Messstelle 24 ist eine photonische Messstelle, worunter hier sämtliche photonische Messprozesse, Absorptions- und Lumineszenzmessungen, bezeichnet werden. Vorzugsweise ist die Messstelle 24 zur Lumineszenzmessung insbesondere zur Fluoreszenzmessung C vorgesehen, wie in **Fig. 8** schematisch skizziert ist. Monochromatische Strahlung L_{C1} oder L_{C2} von einer Anregungslichtquelle 17C, die Teil des Handanalysengeräts 1 ist, das nachfolgend noch ausführlich erläutert wird, tritt an der Messstelle 24 durch die im Probenaufnahmeraum 31 aufgenommene Liquidprobe, die eine fluoreszierende Markersubstanz enthält, die kurz nach Anregung durch die Strahlung L_{C1} oder L_{C2} Fluoreszenz F zeigt, aus. Das dabei emittierte Licht ist in der Regel energieärmer, weist also eine größere Wellenlänge auf. Anders als dargestellt, kann der Detektor 18C, der die zur Konzentration der fluoreszierenden Substanz proportionale Strahlungsleistung der Fluoreszenz erfasst, mittels geeigneter optischer Elemente, die dem Fachmann bekannt sind, auch senkrecht zur Achse des einfallenden Lichts angeordnet werden. In **Fig. 8** ist ferner mit den Anregungslichtstrahlen L_{C1} und L_{C2} angedeutet, dass Anregungslicht unterschiedlicher Wellenlängen zur Detektion unterschiedlicher Markersubstanzen verwendet werden können. Beispielsweise kann zur Anregung blaues Licht L_{C1} der Wellenlänge 450 nm und grünes Licht L_{C2} der Wellenlänge 530 nm gewählt werden. So kann auch ein Marker verwendet werden, der aus zwei Farbstoffmolekülen aus der Reihe der Rylenfarbstoffe wie Perylen und Quaterrylen (beispielsweise Lumogen® F gelb 170, Lumogen® F Pink 285, beide erhältlich von BASF AG, Ludwigshafen, Deutschland), oder einer Kombination von Rhodamincarbonylderivaten und Acridinderivaten (wie beispielsweise ATTO® 612 Q 615 nm und ATTO® 495, 498 nm, beide erhältlich von ATTO-TEC GmbH, Siegen, Deutschland) aufweist, sodass die zwei Messbereiche im langwelligen Bereich abgedeckt werden können.

Alternativ zur Fluoreszenzmessung ist prinzipiell auch eine Phosphoreszenzmessung (mit entsprechend phosphoreszierenden Markersubstanzen) denkbar. Während jedoch Fluoreszenz schnell nach dem Ende der Anregung (meist innerhalb einer Millionstel Sekunde) endet, tritt bei der Phosphoreszenz ein meist längeres bis zu stundenlanges Nachleuchten auf. Außer einer Lumineszenzmessstelle kommt zudem auch eine Absorptionsmessstelle zur Konzentrationsbestimmung bestimmter Substanzen in Frage, aber die Fluoreszenzmessung weist eine höhere Selektivität sowie größere Empfindlichkeit gegenüber der Absorptionsmessung auf.

Die zweite optische Messstelle 25 des Probenaufnahmeelements 20 ist im vorliegenden Beispiel zur Messung des Brechungsindex B ausgebildet, wobei eine der Platten 30,30', nämlich die lichtaustrittsseitige Platte, in diesem als Brechungsindex-Messstelle 25 vorgesehenen Abschnitt auf der Innenseite eine Prismenstruktur 25' aufweist, wie in **Fig. 7** schematisch angedeutet ist. Dieser Abschnitt des Probenaufnahmeelements 20 mit der Prismenstruktur 25' an der Messstelle 25 bildet somit mit den entsprechenden Komponenten des Handgeräts 1 das Refraktometer, das als Lichtquelle 17B energiesparend eine LED nutzen kann, die beispielsweise gelbes Licht L_{B} der Wellenlänge 580 nm emittiert. Alternativ zu einer LED kann beispielsweise auch eine Laserdiode als Lichtquelle genutzt werden. Als Detektor 18B zur Erfassung der Brechung des Lichtstrahls kann ein CCD-Sensor eingesetzt werden. Da der Brechungsindex temperaturabhängig ist, weist das Handgerät 1 zur Kompensation der Temperatureinflüsse ferner einen Temperatursensor 14 auf, der wie alle anderen Messvorrichtungen des Handgeräts 1 über eine entsprechende Kommunikationsleitung 33 mit der Datenverarbeitungseinheit 13 des Handgeräts 1 verbunden ist.

Das Probenaufnahmeelement 20 aus **Fig. 1** zeigt noch zwei weitere Messstellen 26 und 27 zur pH-Messung A und Leitfähigkeitsmessung D. Die Messstelle 16 ist eine optische pH-Messstelle 26, wozu an dieser Stelle im Probenaufnahmeraum 31 zwischen den beiden Platten 30,30' ein indikatorfarbstoffhaltiges Substrat 26' (vgl. **Fig. 6**) eingebracht ist, aus dessen Farbänderung nach Kontakt mit dem zu prüfenden Liquid der pH-Wert optisch ablesbar ist. Als indikatorfarbstoffhaltiges Substrat 26' kommt beispielsweise einfach ein Abschnitt pH-Papier in Frage. Die hierfür vorgesehenen Messkomponenten des Handgeräts 1 können eine RGB-LED als Lichtquelle 17A vorsehen, deren Licht L_{A} das Probenaufnahmeelement 20 vorbei an der pH-Messstelle 26 durchdringt, an Streuvorrichtungen 18A' gestreut und auf das indikatorfarbstoffhaltiges Substrat 26' in der pH-Messstelle 26 zurückgeworfen und dort nur noch die entsprechende Farbwellenlänge reflektiert wird, die dann von einem Farbdetektor 18A erfasst wird und zur Bestimmung des pH-Wertes herangezogen werden kann.

Sämtliche optischen Messkomponenten 17A,B,C und 18A,B,C bilden die optoelektronische Messvorrichtung 12 des Analysengeräts 1 (siehe **Fig. 9**) und können, wie **Fig. 5** andeutet, in einem Einbettungselement 16 angeordnet sein. Auf die Darstellung von für die jeweiligen Messungen A,B,C bekannten optischen Elementen wie Filter, Linsen, Spiegel etc. wird der Übersichtlichkeit wegen verzichtet. Weiter zeigt **Fig. 5** eine Signalvorrichtung 19, die die von den Detektoren und Sensoren 18A,B,C erfassten Signale zumindest weiterleitet. Anders als dargestellt kann auch für jeden Sensor eine einzelne Signalvorrichtung vorgesehen sein. Die Signalvorrichtung 19 ist über die Schnittstelle 5' und die Kommunikationsleitung 33 mit der Datenverarbeitungseinheit 13 verbunden. Nicht dargestellt ist, dass die Lichtquellen 17A,B,C eine entsprechende Anbindung zur Ansteuerung aufweisen können.

In **Fig. 9** ist zudem als Energiequelle zur elektrischen Versorgung sämtlicher Komponenten über Stromleitungen 33' ein Akku 11 dargestellt. Ferner wird dort die Anbindung der in der anderen Halbschale des Gehäuses 2 angeordneten Anzeigevorrichtung 3, die vorzugsweise ein Touchscreen-Display ist, sowie einer (Mikro-)USB-Schnittstelle 5 über entsprechende Kommunikationsleitungen 33 angedeutet. Anstelle oder zusätzlich zu einer (Mikro-)USB-Schnittstelle 5 kann zur Datenübertragung von einem oder auf ein externes Gerät auch ein Speicherkartensteckplatz oder eine Funkschnittstelle (WLAN, Bluetooth®, etc.) vorgesehen sein. Ferner kann die (Mikro-)USB-Schnittstelle zum Aufladen des Ackus 11 genutzt werden.

Die beiden Halbschalen, die das Gehäuse 2 bilden, können beispielsweise durch Steck- oder Schraubverbindungen aneinander gefügt sein und bei Bedarf, etwa zum Austausch des Akkus 11 oder anderer Komponenten, geöffnet werden. Dazu können die Halbschalen auch an einer Längsseite gelenkig verbunden sein, beispielsweis über ein Scharnier, sodass die Steck- oder Schraubverbindungen nur an der anderen Seite vorliegen müssen.

Anders als dargestellt, kann anstelle eines aufladbaren Akkumulators auch eine Batterie als Energiequelle vorgesehen sein, die zum vereinfachten Austausch in bekannter Weise in einem separaten, mit einem werkzeuglos öffenbaren Gehäuseabschnitt verschlossenen Fach untergebracht ist, das Kontaktmittel für die Batterien aufweist.

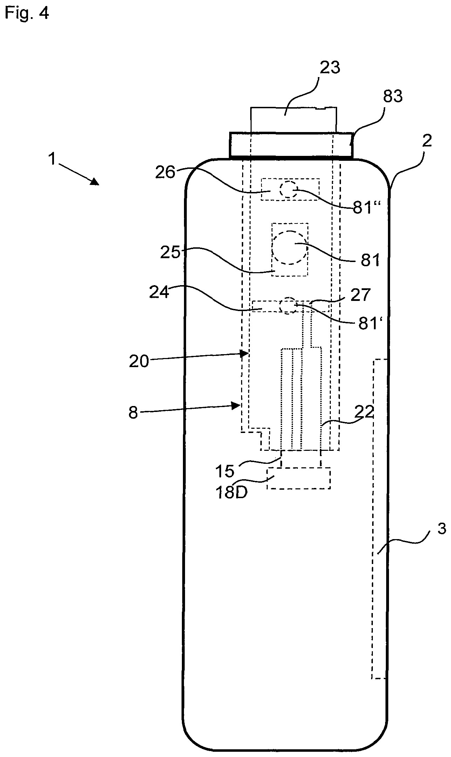

Zur Messung der Leitfähigkeit D der Liquidprobe sind an dem Ende des Probenaufnahmeelements 20, das von dem Griffabschnitt 23 abgewandt ist, Kontaktstreifen 22 auf einem Abschnitt 30" der Basisplatte 30 angeordnet, der längs über das Ende der Abdeckplatte 30' hinausragt. Die freiliegenden Enden dieser Kontaktstreifen 22 können nach Einschieben des Probenaufnahmeelements 20 in das Analysengerät 1 mit entsprechenden Kontaktelementen 15 des Analysengeräts 1 (vgl. **Fig. 4**) in elektrisch leitenden Kontakt kommen, sodass durch einen Frequenzgenerator 18D eine Wechselspannung an den Messenden der Kontaktstreifen 22 anlegbar ist. Die Messenden der Kontaktstreifen 22 bilden an der Messstelle 27 die Elektroden und sind eine vorbestimmte Messstrecke s voneinander beabstandet. Bei der Messung handelt es sich eigentlich um eine Widerstandsmessung, aus der sich die Leitfähigkeit des Liquids berechnen lässt.

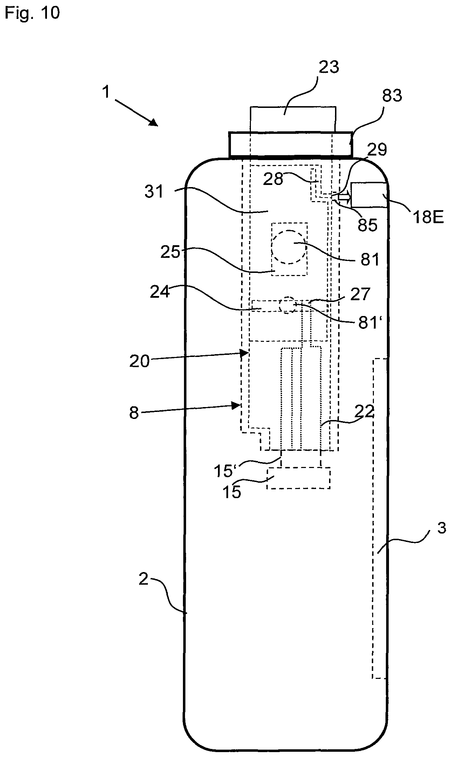

Eine weitere denkbare Messstelle des Probenaufnahmeelements 20 könnte eine Keimmessstelle sein. Ein Beispiel zur Keimmessung ist in **Fig. 10** dargestellt. Hierbei wird der Entlüftungskanal 28 so auf dem Probenaufnahmeelement 20 platziert, dass die Luftaustrittsöffnung 29 sich nicht im Griffabschnitt 23 befindet, sondern eine Messstelle bildet, die über eine Gaskommunikationseinrichtung 85 mit einem oder mehreren Gassensoren, einer "elektronischen Nase" verbunden ist. Gegebenenfalls kann der Entlüftungskanal Querschnittsveränderungen oder eine Bypass-Zuluftführung aufweisen, um eine die Zuleitung der in der Dampfphase des Liquids vorhandenen Moleküle zu der elektronischen Nase zu verbessern. Das Analysegerät kann hierzu beispielsweise eine Lüftervorrichtung aufweisen. Alternativ zum Entlüftungskanal 28 kann auch der vorhandene Befüllspalt als Keimmessstelle für die "elektronische Nase" genutzt werden. Ein anderer Ansatz könnte eine Keimmessstelle sein, bei der zumindest eine der Platten 30,30' einen Abschnitt aus einer gaspermeable Membran aufweist, durch die das Liquid zurückgehalten wird, flüchtige Verbindungen aber durchtreten und so zur "elektronischen Nase" gelangen können. Bei diesen flüchtigen organischen Verbindungen handelt es sich um Absonderungen der Bakterien bzw. Keime. Eine "elektronische Nase" besteht beispielsweise aus mit verschiedenen leitfähigen Polymeren beschichteten Sensoren, die spezifisch auf verschiedene flüchtige Verbindungen reagieren, indem sich ihr elektrischer Widerstand bei Kontakt mit diesen Verbindungen in charakteristischer Weise verändert.

Alternativ zur "elektronischen Nase" können auch Keime mittels einer Lumineszenzmesszelle erfasst werden, wenn dem Liquid ein Luciferin/Luciferase-Gemisch zugegeben wird, das mit Adenosin-Tri-Phosphat, das in jeder lebenden Zelle vorkommt, reagiert. Das dabei abgegebene Licht kann ebenfalls mit dem Luminometer gemessen werden und ist ein Maß für die mikrobiologische Kontamination des Liquids.

Je nach Zusammensetzung des Liquids kommt als weitere Methode zur Keimbestimmung auch eine UV-Absorptionsmessung in Frage, da Nukleinsäuren im UV-Bereich absorbieren.

Die Platten 30,30' sind zumindest im Bereich der Messstellen 24,25,26, an denen eine optische Mess-Sensorik zur Anwendung kommt, zumindest für die entsprechenden Weilenlängen transparent - der einfacheren Fertigung wegen werden die Platten 30,30' komplett aus transparentem Material sein, das Glas, vorzugsweise Quarzglas, oder ein transparenter Kunststoff sein kann. Besonders geeignet sind transparente Kunststoffe wie PMMA. Der Fachmann kennt geeignete Kunststoffe, die sich geeigneter Weise einfach durch 3D- Druck oder Fließpressen herstellen lassen.

Außer der gewünschten Transparenz sollte der Kunststoff zumindest für die Zeitdauer der Probennahme und Analyse chemisch beständig gegen die Inhaltsstoffe des aufzunehmenden Liquids und vorzugsweise auch, sofern das Probenaufnahmeelement 20 eine Leitfähigkeitsmessstelle aufweist, elektrisch isolierend sein. Ist der Kunststoff nicht ausreichen elektrisch isolierend, können die Kontaktstreifen 22 bis zum Probenaufnahmeraum 31 in ein isolierendes Material eingebettet werden. Ein transparenter Kunststoff, der ein guter Isolator ist und beständig gegenüber wässrigen Lösungen von neutralen Salzen und Oxidationsmitteln sowie gegenüber vielen Ölen und Fetten. Allerdings sind Polycarbonate unbeständig gegenüber chlorierten Kohlenwasserstoffen und alkalisch wässrigen Lösungen, Aminen und Ammoniak. Ein anderer transparenter Kunststoff ist Polymethylmethacrylat, welches beständig gegen Säuren, Laugen mittlerer Konzentration, Benzin und Öl ist, nicht jedoch gegen Ethanol, Aceton und Benzol. Polysulfon ist ebenfalls transparent im sichtbaren Bereich, aber unbeständig gegen Ketone, Aromaten, chlorierte Kohlenwasserstoffe und polare Lösungsmittel. Polymethylpenten weist eine sehr hohe Transparenz, auch im UV-Bereich auf, ist aber nicht dauerhaft chemisch beständig gegen Ketone oder chlorierte Lösungsmittel.

Der Griffabschnitt 23, der bei Aufnahme des Probenaufnahmeelements 20 in dem Analysengerät zumindest teilweise aus dem Analysengerät 1 herausragt, kann opak sein - als eingefärbter Abschnitt zumindest einer der Platten 30,30' oder als angefügter Griffabschnitt aus geeignetem Material, beispielsweise einem Kunststoff. Vorzugsweise kann der Griffabschnitt 23 schwarz eingefärbt sein, um Streulichteinfall zu verhindern bzw. minimieren. Es ist aber auch denkbar, unterschiedliche Probenaufnahmeelemente 20, die sich in Art oder Verwendungszweck unterscheiden, durch unterschiedlich gefärbte und/oder geformte Griffabschnitte 23 zu kennzeichnen.

Weiter können am Griffabschnitt 23 oder an anderen Stellen des Probenaufnahmeelements 20 Markierungen angebracht sein, um einem Benutzer das richtige Einsetzen des Probenaufnahmeelements 20 in das Analysengerät 1 anzuzeigen und zu erleichtern. Zum gleichen Zweck kann das Probenaufnahmeelement 20 an dem vom Griffabschnitt 23 abgewandten Ende (mit den Kontaktstreifen 22) einen in Bezug auf die Längsachse des Probenaufnahmeelements 20 unsymmetrischen Rückschnitt 6 haben, sodass das Probenaufnahmeelement nach dem Schlüssel-Schloss-Prinzip nur in einer Ausrichtung korrekt bis zu einem Anschlag in das Analysengerät 1 eingeführt werden kann, damit die Messstellen 24,25,26,27 mit den jeweiligen Messvorrichtungen kommunizieren können.

Selbstverständlich fallen auch in Form und Anordnung von den dargestellten Beispielen abweichende Ausführungen unter den Schutzumfang der Erfindung. So kann ein Probenaufnahmeelement auch eine von der annährend rechteckigen Form abweichende Form haben; allerdings ist diese Form günstig für die platzsparende Anordnung der Messstellen und der zur Messung erforderlichen Komponenten in dem Analysengerät.

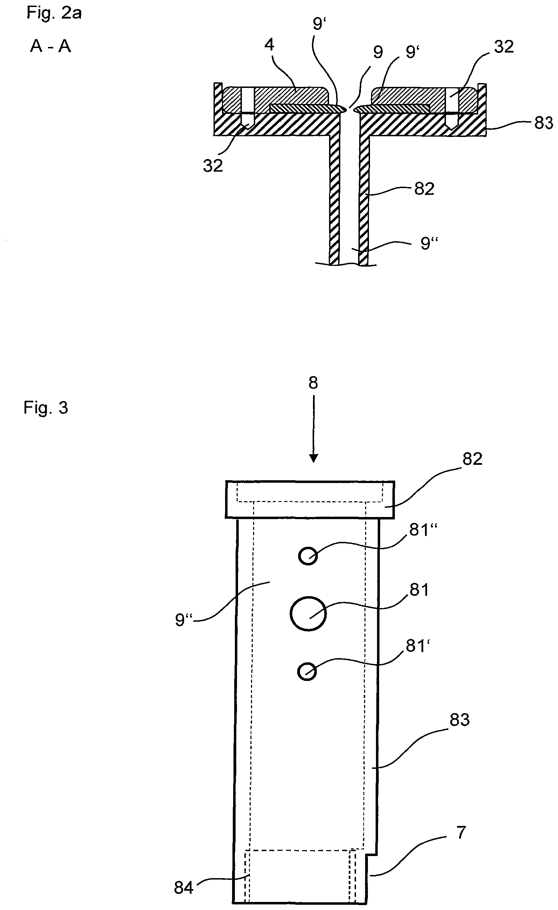

Selbstverständlich ist eine Ausführungsform des erfindungsgemäßen Analysenvorrichtungs-Sets denkbar, in der ein Probenaufnahmeelement 20 direkt in einer entsprechend dimensionierten Ausnehmung eines Analysengerät 1 aufgenommen werden kann. Erfindungsgemäß ist hierfür aber vorteilhaft eine Einsteckvorrichtung 8 vorgesehen, wie sie in **Fig. 2 bis 5****,** **9** **und** **10** zu sehen ist.

Die Einsteckvorrichtung 8 ist lösbar in dem Analysengerät 1 befestigt, sodass sie bei Bedarf ausgetauscht werden kann. Die Einsteckvorrichtung 8 besteht aus einem Hüllabschnitt 82, der sich im Inneren des Analysengeräts 1 erstreckt, und einem Flanschabschnitt 83, der außen am Rand des Gehäuses 2 des Analysengeräts 1 zur Anlage kommt. Im Flanschabschnitt 83 befindet sich eine schlitzartige Einstecköffnung 9, von der sich die Ausnehmung 9" für das Probenaufnahmeelement 20 durch den Hüllabschnitt 82 erstreckt. Dieser weist, wie in **Fig. 3** zu sehen ist, einen zum Rückschnitt 6 des Probenaufnahmeelements 20 korrespondierenden Rückschnitt 7 auf. Ebenfalls korrespondierend zu dem Probenaufnahmeelement 20 und auch zu den Messvorrichtungen im Analysengerät 1 sind als optische Kommunikationseinrichtungen 81,81',81" Öffnungen oder transparente Abschnitte in beiden Seiten des ansonsten opaken, vorzugsweise schwarzen Hüllabschnitts 82 vorgesehen, was auch hier Streulichteffekte verhindern bzw. verringern soll.

Im Flanschabschnitt 83, der im Schnitt auch in **Fig. 2a** dargestellt ist, hält eine Abdeckplatte 4 eine Dichtlippe 9' aus Silikon an der Einstecköffnung 9. Durch die Abdeckplatte 4 und den dazu parallelen Abschnitt des Flanschabschnitts 83 erstrecken sich Bohrungen 32, die ein Verschrauben der Abdeckplatte 4 an dem Flanschabschnitt 83 erlauben. Anders als dargestellt, können die Bohrungen 32 im Flanschabschnitt 83 auch Durchgangsbohrungen sein, sodass nicht nur die Abdeckung 4 am Flanschabschnitt 83, sondern dieser auch am Rand des Gehäuses 2 des Analysengeräts 1 mittels Schrauben lösbar befestigt werden kann. Alternativ kann die Einsteckvorrichtung 8 auch einfach zum Einstecken/Einrasten in das Analysengerät 1 ausgebildet sein.

Die Dichtlippen 9' verhindern eine Kontamination der Ausnehmung 9" im Hüllabschnitt 82. Die Einsteckvorrichtung 8 selbst wiederum verhindert, dass der Innenraum des Analysengeräts 1 verunreinigt oder kontaminiert wird.

Weiter kann vorgesehen sein, dass das Analysengerät 1 eine (nicht gezeigte) öffenbare Abdeckung aufweisen, mit der die Einstecköffnung 9 zusätzlich abgedeckt werden kann. Eine solche Abdeckung lässt sich auch bei eingestecktem Probenaufnahmeelement schließen, sodass hierdurch Streulichteinfall verhindert werden kann und somit auf eine Einfärbung des Griffabschnitts verzichtet werden kann.

Weiter kann die Einsteckvorrichtung 8, an dem von dem Flanschabschnitt 83 abgewandten Ende, in bzw. an dem der Abschnitt 30" des Probenaufnahmeelements 20 mit den Kontaktstreifen 22, wenn das Probenaufnahmeelement 20 in die Ausnehmung 9" der Einsteckvorrichtung 8 eingesteckt ist, elektrische Überbrückungselemente aufweisen, die einen elektrischen Kontakt zwischen den Kontaktstreifen 22 des Probenaufnahmeelements 20 und den Kontaktelementen 15 des Frequenzgenerators 18D herstellen, oder das Einsteckelement 8 weist dort eine als Buchse ausgeformte Aufweitung 84 auf, in die die steckerartig ausgebildete Kontaktelemente 15 des Frequenzgenerators 18D aufgenommen werden können, sodass ein direkter elektrischer Kontakt zwischen den Kontaktstreifen 22 des Probenaufnahmeelements 20 und den Kontaktelementen 15 des Frequenzgenerators 18D hergestellt wird.

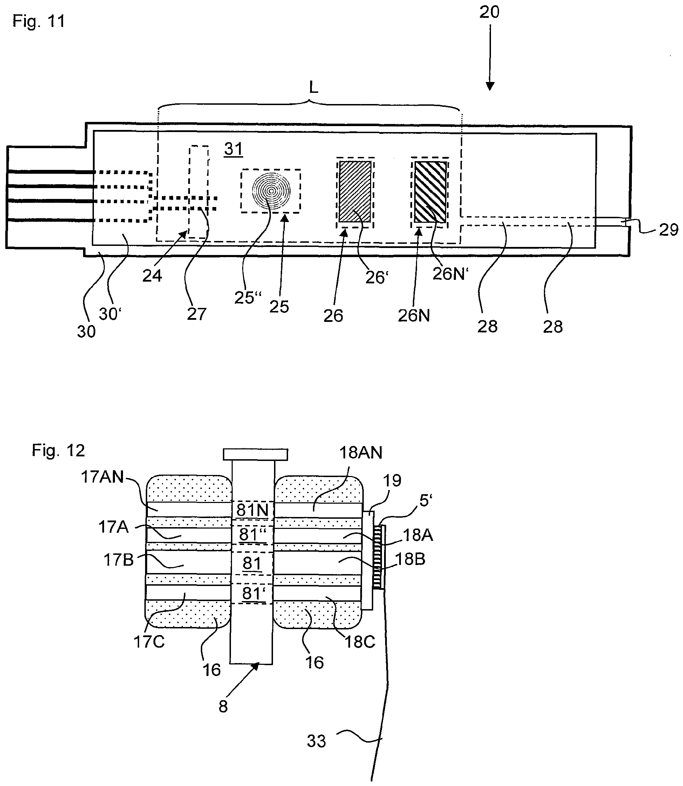

Eine alternative Ausführungsform eines Probenaufnahmeelements 20 ist in **Fig. 11** gezeigt. Zusätzlich zu den bereits beschriebenen Messstellen 24,25 26 und ggf. 29 zur Leitfähigkeits-, Brechungsindex-, pH- und ggf. Keimmessung weist das Probenaufnahmeelement 20 in diesem Beispiel noch eine Nitrit-Messstelle 26N auf, wobei es sich um einen vorbestimmten Abschnitt zwischen den Platten 30,30' handelt, in dem ein nitritreaktives Substrat 26N' eingebracht ist, das eine beispielsweise photometrisch nachweisbare und mit Kalibrierung quantifizierbare Reaktion mit Nitrit ausführt, sodass ähnlich wie bei der pH-Messstelle 26 eine Farbänderung optoelektronisch erfasst werden kann, durch die Vorhandensein und Menge von Nitrat in dem beprobten Liquid nachgewiesen werden kann.

Weiter unterscheidet sich das Probenaufnahmeelement 20 aus **Fig. 11** in der Art der zur Bestimmung des Brechungsindex eingesetzten Prismenstruktur der Messstelle 25. Während die im Zusammenhang mit dem Beispiel nach **Fig. 7** beschriebene Prismenstruktur 25' aus mehreren, benachbart angeordneten, im Profil dreieckigen Strukturen, beispielsweise pyramidalen oder tetraedrischen Strukturen oder auch parallel verlaufenden Dreiecksprofilen auf der Innenseite der lichtaustrittsseitige Platte 30,30' bestehen kann, weist die Brechungsindex-Messstelle 25 des Probenaufnahmeelements 20 aus **Fig. 11** eine Fresnelsche Linsenstruktur 25" als Sonderform einer Prismenstruktur auf, die - ebenfalls auf der Innenseite der lichtaustrittsseitige Platte 30,30' - aus einer Reihe ringförmiger Stufen besteht.

In **Fig. 12** ist ein zum Probenaufnahmeelement 20 aus **Fig. 11** passendes Einsteckelement 8, eingesteckt in das Einbettungselement 16 eines nicht weiter dargestellten Analysengeräts, gezeigt. Im Unterschied zu dem in **Fig. 5** gezeigten Beispiel weist das Einsteckelement 8, korrespondierend zu der zusätzlichen Nitrit-Messstelle 26N des Probenaufnahmeelements 20, wie es in **Fig. 11** gezeigt ist, als optische Kommunikationseinrichtungen 81N eine weitere Öffnung oder einen transparenten Abschnitte in beiden Seiten des ansonsten opaken, vorzugsweise schwarzen, Hüllabschnitts 82 auf, um auch hier Streulichteffekte zu verhindern bzw. zu verringern. Zur Messung des Nitritgehalts ist in das Einbettungselement 16 an entsprechender Stelle eine Messoptik 17AN, 18AN eingebaut, die der Messvorrichtung zur pH-Messung 17A, 18A entsprechen kann. Auch hier können eine RGB-LED als Lichtquelle 17AN und ein Farbdetektor 18AN eingesetzt werden.

Auch das von dem Sensor bzw. Farbdetektor 18A erfasste Signal wird durch die Signalvorrichtung 19 über die Schnittstelle 5' und die Kommunikationsleitung 33 an die Datenverarbeitungseinheit übertragen.

Die Messkomponenten des Refraktometers können unverändert bleiben und beispielsweise durch eine (LED- oder Laserdioden-)Lichtquelle 17B und einen CCD-Sensor 18B gebildet sein.

Ein wesentlicher Punkt der Erfindung ist, dass die Konzentration des Fertigungsmediums bzw. Kühlschmierstoffs in der Emulsion über eine interne Markersubstanz, d. h. einen Farbstoff verfügt, der nach Anregung mit Licht geeigneter Wellenlänge Fluoreszenz zeigt. Ergänzend werden gleichzeitig mit dem einzelnen Probenaufnahmeelement noch der Leitfähigkeitswert, der pH-Wert und der Brechungsindex, aus dem auch Rückschlüsse auf die Konzentration gezogen werden können, in einem einzigen Analysengerät, das als tragbares Handgerät ausgeführt ist, mit einer einzigen Probennahme in einem Messvorgang gemessen.

Anders als große stationäre Analysensysteme, die zwar auch eine große Kenndatenbandbreite erfassen können, aber wirtschaftlich nur bei einer großen Anzahl gleichartiger Proben arbeiten können, ermöglicht das erfindungsgemäße Analysenvorrichtungs-Set auch den wirtschaftlichen Einsatz bei unterschiedlichen speziellen Emulsionen, die nur in begrenztem Umfang, etwa bei Kleinserien, für deren Bearbeitung besondere Anforderungen bestehen, hergestellt werden. So kann das Analysengerät, dessen Luminometer Laser mit (zumindest) zwei unterschiedlichen Anregungswellenlängen aufweist, nicht nur bei einem konventionellen Kühlschmierstoff, dem eine Markersubstanz zugesetzt wurde, bzw. bei einer entsprechenden Emulsion zur Konzentrationserfassung eingesetzt werden, sondern auch bei einem sogenannten "Two Pack System". Dabei wird einer konventionellen Kühlschmierstoffemulsion in der Fertigung zwecks Leistungserhöhung ein Boosteradditiv, meist in einer Konzentration kleiner als etwa 5 Gew.-% zugesetzt, was insbesondere dann der Fall ist, wenn beispielsweise Kleinserien mit besonderen Qualitätsanforderungen in den Standardfertigungsprozess eingeschleust werden müssen. Dann reicht der im Einsatz befindliche konventionelle Kühlschmierstoff leistungsmäßig nicht aus, sodass als Konsequenz die Werkzeugmaschine(n) auf einen anderen Kühlschmierstoff mit höherer Leistungsperformance umgerüstet werden müsste, was zu erhöhter Sortenvielfalt an Kühlschmierstoffen führen würde und unwirtschaftlich wäre. Daher wird in solchen Fällen dem konventionellen Kühlschmierstoff das Boosteradditiv, das dem Kühlschmierstoff vorteilhafte Zusatzeigenschaften beispielsweise im Hinblick auf Dispergiervermögen, Verschieißschutz und/oder Reibwertveränderung verleiht. Allerdings ist bei Zugabe eines Boosteradditivs zur Qualitätssicherung die Überprüfung der Konzentration desselben wichtig, wobei allerdings nur die im Boosteradditiv enthaltenen Inhaltsstoffe und nicht die des Kühlschmierstoffes erfasst werden sollen.

Bislang konnten Boosterkonzentrationsmessungen nur aufwändig im Labor mittels Infrarotspektroskopie und Röntgenfluoreszenzanalyse realisiert werden. Es hat sich nun aber überraschend herausgestellt, dass es bei gezielter Auswahl der Markersubstanz möglich ist, den Booster so zu "dotieren", dass diese Markersubstanz nicht in den Basiskühlschmierstoff "eindiffundiert". Theoretisch liegen somit quasi parallel zwei verschiedene Emulsionssysteme vor, wobei die Konzentrationsbestimmung des Basiskühlschmierstoffs über eine erste Markersubstanz und die Konzentrationsbestimmung des Boosteradditivs über die zweite Markersubstanz erfolgt. Somit ist vor Ort eine eindeutige Konzentrationsbestimmung auch des Boosteradditivs möglich, was bisher nicht realisiert werden konnte.

Selbstverständlich kann die Konzentrationsbestimmung des Kühlschmierstoffs und des Boosteradditivs, denen jeweils eine Markersubstanz zugesetzt wurde, in einer Emulsion mittels Fluoreszenzmessung der unterschiedlichen Markersubstanzen auch durch andere Analysenvorrichtungen als das erfindungsgemäße Analysenvorrichtungs-Set durchgeführt werden - dieses bietet jedoch vorteilhaft kostengünstig die schnelle Analyse direkt vor Ort.

Ein mit dem erfindungsgemäßen Analysenvorrichtungs-Set durchzuführender Messvorgang kann beispielsweise wie folgt ablaufen:
Nach dem Anschalten des Analysengeräts 1, was auf eine übliche Weise durch Gedrückthalten einer farblich markierten Taste an dem Gehäuse 2 erfolgen kann, wird das Touchscreen-Display 3 aktiv - gegebenenfalls kann auch eine Kontroilleuchte neben der Taste aufleuchten - und es erscheint auf dem Display 3 ein Auswahlmenü mit verschiedenen untersuchbaren liquiden Medien, insbesondere Kühlschmierstoffemulsionen, die in einer Datenbank hinterlegt sind, die in der Datenverarbeitungseinheit oder auf einem damit verbundenen Speichermedium (z. B. festes oder Wechselspeichermedium) gespeichert ist. Das zu untersuchende Liquid kann durch Berührung auf dem Touchscreen-Display 3 ausgewählt werden.

Die Probennahme des zu untersuchenden Liquids kann durch Eintauchen des Probenaufnahmeelements 20 mit der Befüllöffnung in das Liquid erfolgen - bzw. es genügt das Kontaktieren der Liquidoberfläche mit der Befüllöffnung - wobei sich der Probenaufnahmeraum 31 durch Kapillarwirkung mit dem Liquid füllt. Die Zeitdauer hierfür beträgt üblicherweise einige Sekunden und kann in Abhängigkeit der gewählten Dimensionen des Probenaufnahmeelements 20 variieren, bis der Probenaufnahmeraum 31 vollständig durch den Kapillareffekt mit dem Liquid befüllt ist, wobei vorhandene Luft durch den Entlüftungskanal 28 entweichen kann.

Die Kühlschmierstoffemulsion bzw. das Liquid sollte bei Probennahme gut durchmischt sein. Daher ist gegebenenfalls vor Probennahme das Liquid zu durchmischen, um eine homogene Verteilung des Kühlschmierstoffs in der Emulsion sicherzustellen. Alternativ zum Eintauchen bzw. Halten an die Liquidoberfläche kann auch eine Pipette oder ein ähnliches Probennahmemittel verwendet werden, um eine Probe des Liquids zu ziehen, die dann an der Befüllöffnung in den Probenaufnahmeraum 31 des Probenaufnahmeelements 20 eingefüllt wird. Falls durch das Eintauchen oder Einfüllen Liquid an der Außenfläche des Probenaufnahmeelements 20 haftet, werden diese oder andere Verunreinigungen vor Einstecken des Probenaufnahmeelements 20 in das Analysengerät 1 entfernt.

Das am Griffabschnitt 23 gehaltene Probenaufnahmeelement 20 wird mit dem Abschnitt 30" voraus durch die Einstecköffnung 9 in die als Messkanal ausgebildete Ausnehmung 9" eingesteckt. Die Dichtlippe 9' an der Einstecköffnung 9 verhindert eine Kontamination des Messkanals, der durch den Hüllabschnitt 82 der bei Bedarf austauschbaren Einsteckvorrichtung 8 umgeben ist, die verhindert, dass der Innenraum des Analysengeräts kontaminiert werden kann.

Bei Vollendung des Einsteckvorgangs, wenn die Kontaktstreifen 22 am verlängerten Abschnitt 30" des Probenaufnahmeelements 20 die Kontaktelemente 15 des Analysengeräts1 kontaktieren, wird der Messprozess automatisch gestartet. Ist ein automatischer Start des Messprozesses nicht erwünscht, kann hierfür auch eine Nutzereingabe, etwa Drücken einer entsprechenden auf dem Touchscreen-Display 3 angezeigten Meldung vorgesehen werden.

Nach Beendigung der Messung(en) wird auf dem Touchscreen-Display 3 eine Aufforderung angezeigt, das Probenaufnahmeelement 20 zu entfernen. Ist dies erfolgt, werden die Messwerte angezeigt. Das als Einmal-Teststreifen konzipierte Probenaufnahmeelement 20 kann der Entsorgung zugeführt werden. Es ist im Prinzip zwar denkbar, dass die beiden Platten 30,30', die das Probenaufnahmeelement konstituieren, voneinander gelöst werden können, um den Innenraum zu reinigen und das pH-Indikatorsubstrat zu erneuern, aber unwirtschaftlich.

Die Messwerte können in der Datenverarbeitungseinheit 13 und/oder in einem damit verbundenen Speichermedium gespeichert werden. Weiter können die Messwerte mittels drahtloser Funkverbindung, z. B. nach dem Bluetooth® Standard auf eine externe Datenverarbeitungseinrichtung bzw. einen Speicher übertragen werden. Hierzu wird auf dem Touchscreen-Display 3 ein entsprechend gekennzeichnetes Feld angezeigt, durch dessen Betätigung eine voreingestellte Funkverbindung aufgebaut wird und die Messwerte übertragen werden. Nach Beendigung der Datenübertragung wird diese Verbindung automatisch getrennt oder kann durch erneute Benutzereingabe beendet werden.

Das Ausschalten des Analysengeräts 1 kann, wie das Anschalten, das Drücken der Taste für eine vorbestimmte Zeitdauer, bzw. bis die Kontroilleuchte erlischt, erfordern; es kann aber auch eine automatische Abschaltung nach einem eingestellten Timer erfolgen.

Mit dem Analysengerät 1 können nicht nur die bekannten Liquide analysiert werden, die in der Datenbank hinterlegt sind, sondern es kann auch eine Kalibrierung durchgeführt werden und es können neue Liquide/Kühlschmierstoffe eingelernt werden, die dann in die Datenbank hinzugefügt werden.

Zur Kalibrierung ist durch den Benutzer im Startmenü auf dem Touchscreen-Display 3 ein entsprechend gekennzeichnetes Feld zu betätigen, woraufhin ein Kafibrierungsmenü geöffnet wird, das zur Kalibrierung des Analysengerätes für die Messung der untersuchbaren Parameter Brechungsindex, pH-Wert und Leitfähigkeit entsprechende Bedienfelder aufweist. Zu Kalibrierungszwecken weist das Analysenvorrichtungs-Set unterschiedliche Kalibrierlösungen beispielsweis in Pipettenfläschchen, die in einer separaten Box bereitgestellt werden, auf.

Weiter weist das Kalibrierungsmenü entsprechend gekennzeichnete Bedienfelder auf, durch deren Betätigung neue mit einer Markersubstanz markierte Liquide/ Kühlschmierstoffe eingelernt werden bzw. bereits eingelesene Medien nachkalibriert werden können. Hierzu sind entsprechende fluoreszenzmarkierte Liquide/Kühlschmierstoffe erforderlich. Zur Nachkalibration kann das Analysenvorrichtungs-Set eine Demonstrationslösung mit einem fluoreszenzmarkierter Kühlschmierstoff bereitstellen.

Ein erfindungsgemäßes Analysenvorrichtungs-Set, das zur Analyse von Kühlschmierstoff bzw. Kühlschmierstoffemulsionen vorgesehen ist, kann für folgende Messbereiche ausgelegt sein:
- Brechungsindex von 1,333 bis 1,38 (0 bis 30 Brix)
- pH-Wert von 7 bis 10
- Leitfähigkeit von 0.2 bis 6 mS/cm
- Kühlschmierstoffkonzentration in der Emulsion von 0 bis 15 Gew-% oder zumindest im Bereich von 0 bis 10 Gew-%, gegebenenfalls von 0 bis 5 Gew-%

Für ein anderes Liquid kann das Analysenvorrichtungs-Set auch für andere Messbereiche ausgelegt werden.

**BEZUGSZEICHENLISTE**

| | | | |
|---|---|---|---|
| 1 | Analysegerät | 19 | Signalvorrichtung |
| 2 | Gehäuse | 20 | Probenaufnahmeelement |
| 3 | Anzeigevorrichtung | 22 | Kontaktstreifen |
| 4 | Abdeckung | 23 | Griffabschnitt |
| 5,5' | externe/interne Schnittstelle | 24 | Messstelle für Fluoreszenzmessung (Konzentration) |
| 6, 7 | Rückschnitt | 25 | Messstelle für Brechungsindexmessung |
| 8 | Einsteckvorrichtung | 25' | Prismenstruktur |
| 81,81',81",81N | Kommunikationseinrichtung | 25" | Fresnelsche Linsenstruktur |
| 82 | Hüllabschnitt | 26 | Messstelle für pH-Wertmessung |
| 83 | Flanschabschnitt | 26' | Indikatorsubstrat |
| 84 | Buchse/Öffnung für Kontakt | 26N | Nitrit-Messstelle |
| 85 | Gaskommunikationseinrichtung | 26N' | nitritreaktives Substrat |
| 9 | Einstecköffnung | 27 | Messstelle für Leitwertmessung |
| 9' | Dichtlippe | 28 | Kanal |
| 9" | Ausnehmung | 29 | Luftaustrittsöffnung |
| 11 | Akku | 30,30' | Platten |
| 12 | Optoelektronik | 30" | verlängerter Plattenabschnitt für Kontaktstreifen |
| 13 | Datenverarbeitungseinheit | 31 | Probenaufnahmeraum |
| 14 | Temperatursensor | 32 | Bohrung |
| 15 | Kontaktelement | 33 | Kommunikationsleitung |
| 16 | Einbettungselement | 33' | Energieversorgungsleitung |
| 17A,B,C,AN | Lichtquelle | | |
| 18A,B,C,AN | Detektor, Sensor | L | Länge Befüllspalt |
| 18A' | Streuvorrichtung | s | Messstrecke |
| 18D | Frequenz-Generator | | |
| 18E | Gassensor | | |

## Patentansprüche

1. Probenaufnahmeelement (20) für eine Liquidprobe für die simultane Analyse von drei oder mehr chemisch-physikalischen Parametern des Liquids mittels einer Analysenvorrichtung,
wobei das Probenaufnahmeelement (20) einen mit dem Liquid befüllbaren Probenaufnahmeraum (31) aufweist, wobei das Probenaufnahmeelement (20) über den Probenaufnahmeraum (31) verteilt zumindest drei benachbart zueinander angeordnete Messstellen (24,25,26,26N,27) aufweist, wobei zwei der Messstellen (24,25) eine photonische Messstelle (24) und eine Brechungsindex-Messstelle (25) sind und wobei die zumindest eine weitere Messstelle ausgewählt ist aus der Gruppe, die zumindest eine pH-Messstelle (26), eine Leitfähigkeits-Messstelle (27) und eine Keimmessstelle umfasst,
wobei das Probenaufnahmeelement (20) ein flächiges Element (20) ist, das zumindest abschnittsweise doppelwandig ist und zwei planparallel aufeinander angeordnete und miteinander verbundene Platten (30,30') aufweist, wobei der Probenaufnahmeraum (31) als ein Spalt flächig zwischen den zwei Platten (30,30') ausgebildet ist,
wobei die Platten (30,30') zumindest abschnittsweise an ihren Rändern miteinander verbunden sind, wobei eine Öffnung des Probenaufnahmeelements (20) durch die nicht verbundenen Randstellen gebildet wird, und ein Abstand zwischen den Platten (30,30') gerade so groß ist, dass die Liquidprobe zwischen den Doppelwänden (30,30') der Kapillarwirkung unterziehbar ist,
**dadurch gekennzeichnet, dass**
die Messstelle (25) für die Brechungsindexmessung eine Prismenstruktur (25',25") an einer der Platten (30,30') in einem dafür vorbestimmten Bereich aufweist, wobei die Platten (30,30') in dem vorbestimmten Bereich für die zur Brechungsindexmessung verwendeten Wellenlängen transparent sind, wobei die Prismenstruktur (25',25") in Bezug auf die Plattenebene abgewinkelte Flächenabschnitte bereitstellt, an denen auftreffende Lichtstrahlen entsprechend gebrochen werden.

2. Probenaufnahmeelement (20) nach Anspruch 1,
**dadurch gekennzeichnet, dass**
die Platten (30,30') zumindest entlang einer Seite, bevorzugt entlang einer langen Seite, nicht miteinander verbunden sind, sodass eine Befüllöffnung oder ein Befüllspalt mit einer Länge (L) für das Liquid bereitgestellt wird.

3. Probenaufnahmeelement (20) nach Anspruch 1 oder 2,
**dadurch gekennzeichnet, dass**
das flächige Element (20) zumindest teilweise aus lichtdurchlässigem Glasmaterial oder einem transparenten Kunststoff besteht.

4. Probenaufnahmeelement (20) nach zumindest einem der Ansprüche 1 bis 3,
**dadurch gekennzeichnet, dass**
die Länge einer der beiden Platten (30) zumindest einenends größer ist als die Länge der anderen Platte (30') und einen Abschnitt (30") aufweist, auf dem zumindest zwei Kontaktstreifen (22) zur Spannungsbeaufschlagung angeordnet sind, die sich in den Probenaufnahmeraum (31) erstrecken und dort durch eine Messstrecke (s) voneinander beabstandet enden, die die Messstelle (27) für die Leitfähigkeitsmessung bildet.

5. Probenaufnahmeelement (20) nach Anspruch 4,
**dadurch gekennzeichnet, dass**
an einem von dem Ende mit den Kontaktstreifen (22) abgewandten anderen Ende das flächige Element (20) als ein Griffabschnitt (23) zur Handhabung des Probenaufnahmeelements (20) ausgebildet ist und dass sich bevorzugt von der Befüllöffnung oder dem Befülispalt mit der Länge (L) ein Fluidpfad entlang der Messstellen (24,27,25,26,26N) bis zu einem Entlüftungskanal (28) erstreckt, der an einer Luftaustrittsöffnung (29) an der Außenseite des flächigen Elements (20) mündet.

6. Probenaufnahmeelement (20) nach zumindest einem der Ansprüche 1 bis 5,
**dadurch gekennzeichnet, dass**
- die photonische Messstelle (24) eine Lumineszenzmessstelle (24) ist, wobei die Platten (30,30') an einem vorbestimmten ersten Abschnitt für die Anregungs- und Emissionswellenlängen der vorgesehenen Lumineszenzmessung transparent sind, und/oder
- die Messstelle für die pH-Wert-Messung (26) ein indikatorfarbstoffhaltiges Substrat (26') aufweist, das an einem vorbestimmten zweiten Abschnitt zwischen den beiden Platten (30,30') angeordnet ist, und/oder
- die Prismenstruktur (25') durch wenigstens eine, vorzugsweise mehrere, benachbart angeordnete, im Profil dreieckige Strukturen oder eine Fresnelsche Linsenstruktur (25"), die eine Reihe ringförmiger Stufen aufweist, gebildet wird, die an einem vorbestimmten dritten Abschnitt einer der beiden Platten (30,30') vorgesehen ist, und/oder
- die Gruppe, aus der die zumindest eine weitere Messstelle ausgewählt ist, zusätzlich eine Nitrit-Messstelle (26N) umfasst, die ein nitritreaktives Substrat (26N') aufweist, das an einem vorbestimmten vierten Abschnitt zwischen den beiden Platten (30,30') angeordnet ist.

7. Analysenvorrichtungs-Set für die simultane Analyse zumindest drei unterschiedlicher chemisch-physikalischer Parameter von Liquiden;
wobei das Analysenvorrichtungs-Set
- ein als Handgerät ausgebildetes Analysegerät (1) mit einem Gehäuse (2) und mit einer Anzeigevorrichtung (3), sowie
- zumindest ein Probenaufnahmeelement (20) für eine Liquidprobe aufweist,
**dadurch gekennzeichnet, dass**
das Probenaufnahmeelement (20) ein Probenaufnahmeelement (20) nach zumindest einem der Ansprüche 1 bis 6 ist,
und das Analysengerät (1) eine optoelektronische Analysenvorrichtung (12) und eine Datenverarbeitungseinheit (13) aufweist, die mit der Analysenvorrichtung (12) und der Anzeigevorrichtung (3) kommunikativ verbunden ist,
wobei die optoelektronische Analysenvorrichtung (12) zumindest drei benachbart zueinander angeordnete Messvorrichtungen (15,17,18) aufweist, deren Anordnung mit der Anordnung der Messstellen (24,25,26,26N,27) auf dem Probenaufnahmeelement (20) korrespondiert.

8. Analysenvorrichtungs-Set nach Anspruch 7,
**dadurch gekennzeichnet, dass**
das Analysengerät (1) eine Einsteckvorrichtung (8) zur Aufnahme des Probenaufnahmeelements (20) aufweist, die lösbar in dem Gehäuse (2) angeordnet ist und eine Einstecköffnung (9) aufweist, die in eine zur Aufnahme des Probenaufnahmeelements (20) korrespondierend dazu ausgebildete Ausnehmung (9") mündet, wobei die Einsteckvorrichtung (8) korrespondierend zu den Anordnungen der Messvorrichtungen (15, 17, 18) und der Messstellen (24,25,26,26N,27) jeweils in Abhängigkeit der Art der jeweiligen Messstelle (24,25,26,26N,27) eine optische, elektronische oder optoelektronische Kommunikationseinrichtung (81,81',81",81N) aufweist.

9. Analysenvorrichtungs-Set nach Anspruch 7 oder 8,
**dadurch gekennzeichnet, dass**
die Einsteckvorrichtung (8) einen Flanschabschnitt (83) mit der Einstecköffnung (9) und einen Hüllabschnitt (82) aufweist, der in dem Gehäuse (2) lösbar angeordnet ist, die Ausnehmung (9") begrenzt und die optischen, elektronischen oder optoelektronischen Kommunikationseinrichtungen (81,81',81",81N) aufweist, die durch Abschnitte aus transparentem Material und/oder durch Öffnungen in dem Hüllabschnitt (82) gebildet sind, der ansonsten aus opakem Material gefertigt ist.

10. Analysenvorrichtungs-Set nach zumindest einem der Ansprüche 7 bis 9,
wobei zwei der Messvorrichtungen (15,17,18) eine photonische Messvorrichtung, bevorzugt eine Lumineszenzmessvorrichtung (17C,18C), und eine Brechungsindex-Messvorrichtung (17B,18B) sind und wobei die zumindest eine weitere Messvorrichtung (15,17,18) ausgewählt ist aus der Gruppe, die zumindest eine pH-Messvorrichtung (17A,18A), eine Leitfähigkeits-Messvorrichtung (15,18D), eine Nitrit-Messvorrichtung (17AN,18AN) und eine Messvorrichtung (18E) zur Erfassung der Keimbelastung umfasst,
- die Lumineszenzmessvorrichtung (17C,18C), die Brechungsindex-Messvorrichtung (17B,18B), die pH-Messvorrichtung (17A,18A) und die Nitrit-Messvorrichtung (17AN,18AN) jeweils eine Lichtquelleneinheit (17A,17B, 17C,17AN) und eine Detektionseinheit (18A, 18B, 18C,18AN) aufweisen, die beidseitig der entsprechenden Messstellen (24,25,26,27,26N) des in dem Analysengerät (1) aufgenommenen Probenaufnahmeelements (20) in dem Gehäuse (2) angeordnet sind.
- dass das Analysengerät (1) eine Temperaturmessvorrichtung (14) aufweist, die mit der Datenverarbeitungseinheit (13) verbunden ist,
- die Leitfähigkeits-Messvorrichtung (15,18D) einen Frequenzgenerator (18D) mit Kontaktelementen (15) aufweist, die bei Anordnung des Probenaufnahmeelements (20) in dem Analysengerät mit den zumindest zwei Kontaktstreifen (22) des Probenaufnahmeelements (20) in elektrischen Kontakt stehen,
- die Messvorrichtung (18E) zur Erfassung der Keimbelastung zumindest ein mikroelektronischer Gassensor (18E) ist, der über eine Verbindungsleitung mit dem Probenaufnahmeraum (31) in Verbindung steht.

11. Analysenvorrichtungs-Set nach zumindest einem der Ansprüche 8 bis 10,
**dadurch gekennzeichnet, dass**
der Flanschabschnitt (83) der Einsteckvorrichtung (8) in einer Analyseanordnung, in der die Einsteckvorrichtung (8) in das Gehäuse (2) eingesteckt ist, an einem Rand des Gehäuses (2) außenseitig anliegt und eine Abdeckplatte (4) einfasst, in die die Einstecköffnung (9) eingebracht ist, die durch eine Dichtlippe (9') abgedichtet wird, die von der Abdeckplatte (4) in dem Flanschabschnitt (83) gehalten wird, wobei die Abdeckplatte (4) lösbar in dem Flanschabschnitt (83) befestigt ist.

12. Analysenvorrichtungs-Set nach Anspruch 10 oder 11,
**dadurch gekennzeichnet, dass**
die Einsteckvorrichtung (8) Kontaktbrücken aufweist, die den Kontakt der Kontaktelemente (15) des Analysengeräts (1) mit den zumindest zwei Kontaktstreifen (22) des Probenaufnahmeelements (20) herstellen.

13. Analysenvorrichtungs-Set nach zumindest einem der Ansprüche 7 bis 12,
**dadurch gekennzeichnet, dass**
das Analysengerät (1) eine Energiequelle, bevorzugt einen Akkumulator (11) aufweist, der in dem Gehäuse (2) angeordnet ist und die Energieversorgung der optoelektronischen Analysenvorrichtung (12), der Datenverarbeitungseinheit (13) und der Anzeigevorrichtung (3) bereitstellt.

14. Analysenvorrichtungs-Set nach zumindest einem der Ansprüche 7 bis 13,
**dadurch gekennzeichnet, dass**
- die Anzeigevorrichtung (3) als Bedienschnittstelle eine berührungssensitive Anzeigevorrichtung (3) ist,
- die Datenverarbeitungseinheit (13) eine externe Kommunikationsschnittstelle (5) aufweist oder damit verbunden ist, wobei die externe Kommunikationsschnittstelle (5) eine Steckkontaktschnittstelle oder eine Funkschnittstelle ist.

15. Verfahren zur simultanen Analyse zumindest dreier unterschiedlicher chemisch-physikalischer Parameter eines Liquids unter Verwendung eines Analysenvorrichtungs-Sets nach zumindest einem der Ansprüche 7 bis 14,
**umfassend die Schritte**
- Eintauchen des Probenaufnahmeelements (20) in das Liquid oder Kontaktieren der Liquidoberfläche mit einer Öffnung des Probenaufnahmeelements (20), die durch die nicht verbundenen Randstellen gebildet wird, und Füllen des Probenaufnahmeraums (31) des Probenaufnahmeelements (20) mit einer Liquidprobe durch die Kapillarwirkung zwischen den Doppelwänden (30,30') des Probenaufnahmeelements (20),
- vollständig Einstecken des Probenaufnahmeelements (20) in das Analysengerät (1),
- Starten und Durchführen von zumindest drei oder mehr Messvorgängen gleichzeitig durch die Mess-Vorrichtungen (18A,B,C,D,E,AN) an den Messstellen (24,25,26,27,28,26N),
- nach Beendigung der Messvorgänge Anzeigen der Messergebnisse auf der Anzeigevorrichtung (3).

16. Verfahren nach Anspruch 15,
**wobei**
- verschiedene untersuchbare Liquide in einer Datenbank hinterlegt sind, die in der Datenverarbeitungseinheit oder auf einem damit verbundenen Speichermedium gespeichert sind, und vor dem Starten und Durchführen von zumindest drei oder mehr Messvorgängen gleichzeitig durch die Mess-Vorrichtungen (18A,B,C,D,E,AN) an den Messstellen (24,25,26,27,28,26N) Auswählen des zu untersuchenden Liquids durch eine Benutzereingabe auf der Anzeigenvorrichtung (3).

17. Verfahren nach Anspruch 15 oder 16,
**umfassend die Schritte**
- automatisch oder nach Benutzereingabe Erfassen des vollständigen Einsteckens des Probenaufnahmeelements (20) in das Analysengerät (1) und/oder
- nach Beendigung der Messvorgänge auf der Anzeigenvorrichtung (3) Anzeigen einer Aufforderung zum Entfernen des Probenaufnahmeelements (20) aus dem Analysengerät (1), und/oder
- nach erfasster Entnahme des Probenaufnahmeelements (20) aus dem Analysengerät (1) Anzeigen der Messergebnisse auf der Anzeigevorrichtung (3) und Speichern und/oder Übertragen der Messergebnisse.

18. Verfahren nach Anspruch 15 oder 16,
**umfassend die Schritte**
- Kalibrieren des Analysengeräts (1) für die untersuchbaren Liquide, die in der Datenbank hinterlegt sind, unter Verwendung von Kalibrierlösungen und/oder
- Einlernen neuer Liquide mit bekannten chemisch-physikalischen Parametern mit dem Analysengerät (1) und Hinzufügen der eingelernten Liquide in die Datenbank.

19. Verfahren nach Anspruch 17 oder 18,
**wobei**
das Liquid zumindest eine mittels Lumineszenzanalyse nachweisbare Markersubstanz aufweist und wobei eine der Messstellen (24,25,26,27,28,26N) eine Lumineszenz-Messstelle (24) ist.

20. Verfahren nach Anspruch 19,
**wobei**
das zu analysierende Liquid eine Metallbearbeitungsflüssigkeit, insbesondere ein Kühlschmierstoff, besonders bevorzugt eine Kühlschmierstoffemulsion ist, wobei dem Liquid zumindest eine erste mittels Lumineszenzanalyse nachweisbare Markersubstanz in einer vorbestimmten Konzentration zugesetzt ist.

21. Verfahren nach Anspruch 20,
**wobei**
das Liquid ein Boosteradditiv aufweist und dem Liquid zumindest eine zweite mittels Lumineszenzanalyse nachweisbare Markersubstanz in einer vorbestimmten Konzentration zugesetzt ist, die sich von der ersten Markersubstanz hinsichtlich ihrer Lumineszenzeigenschaften unterscheidet.

## Claims

1. Sample-holding element (20) for a liquid sample for simultaneous analysis of three or more chemico-physical parameters of the liquid by means of an analysis device, wherein the sample-holding element (20) comprises a sample-holding space (31) that can be filled with the liquid, wherein the sample-holding element (20) comprises at least three measuring points (24, 25, 26, 26N, 27) in adjacent arrangement with respect to each other distributed across the sample-holding space (31), wherein two of the measuring points (24, 25) are a photonic measuring point (24) and a refractive index measuring point (25), and wherein the at least one further measuring point is selected from the group comprising at least one pH measuring point (26), one conductivity measuring point (27), and one germ measuring point;
wherein the sample-holding element (20) is a planar element (20) that is double-walled at least in sections and has two planar-parallel plates (30, 30') arranged on top of each other that are connected to each other, wherein the sample-holding space (31) is designed in the form of a gap in planar manner between the two plates (30, 30');
wherein the plates (30, 30') are connected to each other at least in sections at their edges, wherein an opening of the sample-holding element (20) is formed by the non-connected parts of the edge, and a distance between the plates (30, 30') is just so large that the liquid sample between the double walls (30, 30') can be subjected to the capillary effect,
**characterised in that**
the measuring point (25) for the refractive index measurement comprises a prism structure (25', 25") at one of the plates (30, 30') in an area predetermined for this purpose, wherein the plates (30, 30') are transparent in the predetermined area to the wavelengths used for the refractive index measurement, wherein the prism structure (25', 25") provides surface sections that are angled with respect to the plate plane, at which incident light beams are refracted accordingly.

2. Sample-holding element (20) according to claim 1,
**characterised in that**
the plates (30, 30') are not connected to each other at least along one side, preferably along a long side, such that a filling opening or a filling gap with a length (L) for the liquid is provided.

3. Sample-holding element (20) according to claim 1 or 2
**characterised in that**
the planar element (20) consists, at least in part, of translucent glass material or a transparent plastic material.

4. Sample-holding element (20) according to at least one of the claims 1 to 3,
**characterised in that**
the length of one of the two plates (30) is larger, at least on one end, than the length of the other plate (30') and comprises a section (30") on which at least two contact strips (22) for the application of voltage are arranged and extend into the sample-holding space (31) and end there at a distance from each other that corresponds to a measuring section (s) that forms the measuring point (27) for the conductivity measurement.

5. Sample-holding element (20) according to claim 4,
**characterised in that**
the planar element (20) is designed, at an other end that faces away from the end with the contact strips (22), in the form of a handle section (23) for handling of the sample-holding element (20) and **in that** a fluid path preferably extends from the filling opening or the filling gap of length (L) along the measuring points (24, 27, 25, 26, 26N) to a ventilation channel (28) that terminates at an air exit opening (29) on the outside of the planar element (20).

6. Sample-holding element (20) according to at least one of the claims 1 to 5,
**characterised in that**
- the photonic measuring point (24) is a luminescence measuring point (24), wherein the plates (30, 30') are transparent to the excitation and emission wavelengths of the intended luminescence measurement at a predetermined first section, and/or
- the measuring point for the pH measurement (26) comprises an indicator dye-containing substrate (26') that is arranged at a predetermined second section between the two plates (30, 30'), and/or
- the prism structure (25') is formed by at least one, preferably more, structures with a triangular profile in an adjacent arrangement or a Fresnel lens structure (25") that comprises a series of ring-shaped steps and is provided at a predetermined third section of one of the two plates (30, 30'), and/or
- the group from which the at least one further measuring point is selected comprises, in addition, a nitrite measuring point (26N) that comprises a nitrite-reactive substrate (26N') that is arranged at a predetermined fourth section between the two plates (30, 30').

7. Analysis device set for simultaneous analysis of at least three different chemico-physical parameters of liquids;
wherein the analysis device set comprises
- an analysis apparatus (1) designed as a hand-held device with a housing (2) and with a display device (3), as well as
- at least one sample-holding element (20) for a liquid sample,
**characterised in that**
the sample-holding element (20) is a sample-holding element (20) according to at least one of the claims 1 to 6,
and the analysis apparatus (1) comprises an optoelectronic analysis device (12) and a data processing unit (13) that is connected in communicative manner to the analysis device (12) and the display device (3),
wherein the optoelectronic analysis device (12) comprises at least three measuring devices (15, 17, 18) in an adjacent arrangement with respect to each other, whose arrangement matches the arrangement of the measuring points (24, 25, 26, 26N, 27) on the sample-holding element (20).

8. Analysis device set according to claim 7,
**characterised in that**
the analysis apparatus (1) comprises an insertion device (8) for accommodation of the sample-holding element (20) that is arranged in the housing (2) in detachable manner and comprises an insertion opening (9) that terminates in a recess (9") for accommodation of the sample-holding element (20) that is designed to match it, wherein the insertion device (8) comprises an optical, electronic or optoelectronic communication facility (81, 81', 81", 81N) that matches the arrangements of the measuring devices (15, 17, 18) and measuring points (24, 25, 26, 26N, 27) depending on the type of the respective measuring point (24, 25, 26, 26N, 27).

9. Analysis device set according to claim 7 or 8,
**characterised in that**
the insertion device (8) comprises a flange section (83) with the insertion opening (9) and a shell section (82) that is arranged in the housing (2) such as to be detachable, borders the recess (9") and comprises the optical, electronic or optoelectronic communication facilities (81, 81', 81", 81N) that are formed by sections made of transparent material and/or by openings in the shell section (82), which apart from that is manufactured from opaque material.

10. Analysis device set according to at least one of the claims 7 to 9,
wherein two of the measuring devices (15, 17, 18) are a photonic measuring device, preferably a luminescence measuring device (17C, 18C), and a refractive index measuring device (17B, 18B), and wherein the at least one further measuring device (15, 17, 18) is selected from the group comprising at least one pH measuring device (17A, 18A), one conductivity measuring device (15, 18D), one nitrite measuring device (17AN, 18AN), and one measuring device (18E) for detection of the germ load,
- the luminescence measuring device (17C, 18C), the refractive index measuring device (17B, 18B), the pH measuring device (17A, 18A), and the nitrite measuring device (17AN, 18AN) each comprise a light source unit (17A, 17B, 17C, 17AN) and a detection unit (18A, 18B, 18C, 18AN) that are arranged in the housing (2) on both sides of the corresponding measuring points (24, 25, 26, 27, 26N) of the sample-holding element (20) accommodated in the analysis apparatus (1),
- in that the analysis apparatus (1) comprises a temperature measuring device (14) that is connected to the data processing unit (13);
- the conductivity measuring device (15, 18D) comprises a frequency generator (18D) with contact elements (15), which are in electrical contact with the at least two contact strips (22) of the sample-holding element (20) when the sample-holding element (20) is arranged in the analysis apparatus;
- the measuring device (18E) for detection of the germ load is at least one microelectronic gas sensor (18E) that is connected to the sample-holding space (31) by means of a connecting line.

11. Analysis device set according to at least one of the claims 8 to 10,
**characterised in that**
the flange section (83) of the insertion device (8), in an analytical arrangement, in which the insertion device (8) is inserted into the housing (2), touches, on the outside, against an edge of the housing (2) and frames a cover plate (4), in which the insertion opening (9) has been made, which is sealed by a sealing lip (9') that is held in the flange section (83) by the cover plate (4), wherein the cover plate (4) is fastened in the flange section (83) such as to be detachable.

12. Analysis device set according to claim 10 or 11,
**characterised in that**
the insertion device (8) comprises contact bridges that establish the contact of the contact elements (15) of the analysis apparatus (1) to the at least two contact strips (22) of the sample-holding element (20).

13. Analysis device set according to at least one of the claims 7 to 12,
**characterised in that**
the analysis device (1) comprises an energy source, preferably a rechargeable battery (11), which is arranged in the housing (2) and provides the energy supply of the optoelectronic analysis device (12), of the data processing unit (13), and of the display device (3).

14. Analysis device set according to at least one of the claims 7 to 13,
**characterised in that**
- the display device (3), as a control interface, is a touch-sensitive display device (3),
- the data processing unit (13) comprises or is connected to an external communication interface (5), wherein the external communication interface (5) is a plug contact interface or a radio interface.

15. Method for simultaneous analysis of at least three different chemico-physical parameters of a liquid through the use of an analysis device set according to at least one of the claims 7 to 14,
**comprising the steps of**
- immersing the sample-holding element (20) into the liquid or contacting an opening of the sample-holding element (20) that is formed by the non-connected parts of the edge to the liquid surface, and filling the sample-holding space (31) of the sample-holding element (20) with a sample of the liquid through the action of the capillary effect between the double walls (30, 30') of the sample-holding element (20),
- completely inserting the sample-holding element (20) into the analysis apparatus (1),
- starting and carrying out at least three or more measuring processes simultaneously by means of the measuring devices (18A,B,C,D,E,AN) at the measuring points (24, 25, 26, 27, 28, 26N),
- after completion of the measuring processes, displaying the measuring results on the display device (3).

16. Method according to claim 15,
**wherein**
- various liquids that can be tested are deposited in a database that is stored in the data processing unit or on a storage medium connected to it, and selecting the liquid to be tested through a user input on the display device (3) before starting and carrying out at least three or more measuring processes simultaneously by means of the measuring devices (18A,B,C,D,E,AN) at the measuring points (24, 25, 26, 27, 28, 26N).

17. Method according to claim 15 or 16,
**comprising the steps of**
- detecting the complete insertion of the sample-holding element (20) into the analysis apparatus (1) automatically or after a user input, and/or
- after completion of the measuring processes, displaying on the display device (3) a prompt for removal of the sample-holding element (20) from the analysis apparatus (1), and/or
- after the removal of the sampling-holding element (20) from the analysis apparatus (1) has been detected, displaying the measuring results on the display device (3), and storing and/or transmitting the measuring results.

18. Method according to claim 15 or 16,
**comprising the steps of**
- calibrating the analysis apparatus (1) for the liquids that can be tested and are deposited in the database, through the use of calibration solutions, and/or
- inputting new liquids with known chemico-physical parameters with the analysis apparatus (1) and adding the inputted liquids to the database.

19. Method according to claim 17 or 18
**wherein**
the liquid comprises at least one marker substance that can be detected by means of luminescence analysis, and wherein one of the measuring points (24, 25, 26, 27, 28, 26N) is a luminescence measuring point (24).

20. Method according to claim 19,
**wherein**
the liquid to be analysed is a metal processing liquid, in particular a cooling lubricant, particularly preferably a cooling lubricant emulsion, wherein at least one first marker substance that can be detected by means of luminescence analysis is added to the liquid at a predetermined concentration.

21. Method according to claim 20,
**wherein**
the liquid comprises a booster additive and at least one second marker substance that can be detected by means of luminescence analysis is added to the liquid at a predetermined concentration, wherein the second marker substance differs from the first marker substance with regard to its luminescence properties.

## Revendications

1. Elément de réception d'échantillons (20) pour un échantillon liquide pour l'analyse simultanée de trois paramètres chimiques-physiques ou plus du liquide au moyen d'un dispositif d'analyse,
dans lequel l'élément de réception d'échantillons (20) présente un espace de réception d'échantillons (31) pouvant être rempli du liquide, dans lequel l'élément de réception d'échantillons (20) présente au moins trois points de mesure (24, 25, 26, 26N, 27) disposés de manière avoisinante les uns par rapport aux autres répartis sur l'espace de réception d'échantillons (31), dans lequel deux des points de mesure (24, 25) sont un point de mesure photonique (24) et un point de mesure d'indice de réfraction (25) et dans lequel l'au moins un autre point de mesure est sélectionné parmi le groupe qui comprend au moins un point de mesure de pH (26), un point de mesure de conductivité (27) et un point de mesure germinative,
dans lequel l'élément de réception d'échantillons (20) est un élément plan (20) qui est à double paroi au moins par sections et présente deux plaques (30, 30') disposées l'une sur l'autre à faces planes et parallèles et connectées l'une à l'autre, dans lequel l'espace de réception d'échantillons (31) est réalisé en tant que fente de manière plane entre les deux plaques (30, 30'),
dans lequel les plaques (30, 30') sont connectées l'une à l'autre au moins par sections à leurs bords, dans lequel une ouverture de l'élément de réception d'échantillons (20) est formée par les points de bord non connectés, et un écart entre les plaques (30, 30') est aussi grand que l'échantillon liquide peut être soumis à l'action capillaire entre les doubles parois (30, 30'),
**caractérisé en ce que**
le point de mesure (25) pour la mesure d'indice de réfraction présente une structure en prisme (25', 25") sur une des plaques (30, 30') dans une région prédéterminée à cet effet, dans lequel les plaques (30, 30') sont transparentes dans la région prédéterminée pour les longueurs d'onde utilisées pour la mesure d'indice de réfraction, dans lequel la structure en prisme (25', 25") met à disposition des sections de face coudées par rapport au plan de plaque sur lesquelles des rayons lumineux incidents sont réfractés en conséquence.

2. Elément de réception d'échantillons (20) selon la revendication 1,
**caractérisé en ce que**
les plaques (30, 30') ne sont pas connectées l'une à l'autre au moins le long d'un côté, de préférence le long d'un côté long, de sorte qu'une ouverture de remplissage ou une fente de remplissage avec une longueur (L) est mise à disposition pour le liquide.

3. Elément de réception d'échantillons (20) selon la revendication 1 ou 2,
**caractérisé en ce que**
l'élément plan (20) se compose au moins partiellement de matériau de verre translucide ou d'un plastique transparent.

4. Elément de réception d'échantillons (20) selon au moins une des revendications 1 à 3, **caractérisé en ce que**
la longueur d'une des deux plaques (30, 30') est supérieure au moins à une extrémité à la longueur de l'autre plaque (30') et présente une section (30") sur laquelle au moins deux bandes de contact (22) pour la sollicitation en tension sont disposées, lesquelles s'étendent dans l'espace de réception d'échantillons (31) et s'y terminent de manière écartée l'une de l'autre par un trajet de mesure (s) qui forme le point de mesure (27) pour la mesure de conductivité.

5. Elément de réception d'échantillons (20) selon la revendication 4,
**caractérisé en ce que**
l'élément plan (20) est réalisé en tant que section de poignée (23) pour la manipulation de l'élément de réception d'échantillons (20) à une autre extrémité détournée de l'extrémité avec les bandes de contact (22) et qu'un chemin de fluide s'étend de préférence de l'ouverture de remplissage ou la fente de remplissage avec la longueur (L) le long des points de mesure (24, 27, 25, 26, 26N) jusqu'à un canal de purge (28) qui débouche au niveau d'une ouverture de sortie d'air (29) sur le côté externe de l'élément plan (20).

6. Elément de réception d'échantillons (20) selon au moins une des revendications 1 à 5, **caractérisé en ce que**
- le point de mesure photonique (24) est un point de mesure de luminescence (24), dans lequel les plaques (30, 30') sont transparentes sur une première section prédéterminée pour les longueurs d'onde d'excitation et d'émission de la mesure de luminescence prévue, et/ou
- le point de mesure pour la mesure de valeur de pH (26) est un substrat (26') contenant un colorant indicateur qui est disposé sur une deuxième section prédéterminée entre les deux plaques (30, 30'), et/ou
- la structure en prisme (25') est formée par au moins une, de préférence plusieurs, structures triangulaires en profil, disposées de manière avoisinante ou une structure de lentilles de Fresnel (25") qui présente une série d'échelons annulaires, qui est prévue sur une troisième section prédéterminée d'une des deux plaques (30, 30'), et/ou
- le groupe parmi lequel l'au moins un autre point de mesure est sélectionné comprend en outre un point de mesure de nitrite (26N) qui présente un substrat réactif au nitrite (26N') qui est disposé sur une quatrième section prédéterminée entre les deux plaques (30, 30').

7. Kit de dispositif d'analyse pour l'analyse simultanée d'au moins trois paramètres chimiques-physiques différents de liquides,
dans lequel le kit de dispositif d'analyse présente
- un appareil d'analyse (1) réalisé en tant qu'appareil portatif avec un logement (2) et avec un dispositif d'affichage (3), ainsi
- qu'au moins un élément de réception d'échantillons (20) pour un échantillon liquide,
**caractérisé en ce que**
l'élément de réception d'échantillons (20) est un élément de réception d'échantillons (20) selon au moins une des revendications 1 à 6,
et l'appareil d'analyse (1) présente un dispositif d'analyse opto-électronique (12) et un module de traitement des données (13) qui est connecté de manière communicative au dispositif d'analyse (12) et au dispositif d'affichage (3),
dans lequel le dispositif d'analyse opto-électronique (12) présente au moins trois dispositifs de mesure (15, 17, 18) disposés de manière avoisinante les uns par rapport aux autres dont l'agencement correspond à l'agencement des points de mesure (24, 25, 26, 26N, 27) sur l'élément de réception d'échantillons (20).

8. Kit de dispositif d'analyse selon la revendication 7,
**caractérisé en ce que**
l'appareil d'analyse (1) présente un dispositif d'insertion (8) pour la réception de l'élément de réception d'échantillons (20) qui est disposé de manière amovible dans le logement (2) et présente une ouverture d'insertion (9) qui débouche dans un évidement (9") réalisé pour la réception de l'élément de réception d'échantillons (20) de manière correspondante à celui-ci, dans lequel le dispositif d'insertion (8) présente une installation de communication (81, 81', 81", 81N) optique, électronique ou opto-électronique correspondant aux agencements des dispositifs de mesure (15, 17, 18) et des points de mesure (24, 25, 26, 26N, 27) à chaque fois en fonction du type du point de mesure respectif (24, 25, 26, 26N, 27).

9. Kit de dispositif d'analyse selon la revendication 7 ou 8,
**caractérisé en ce que**
le dispositif d'insertion (8) présente une section de bride (83) avec l'ouverture d'insertion (9) et une section d'enveloppe (82) qui est disposée de manière amovible dans le logement (2), limite l'évidement (9") et présente les installations de communication (81, 81', 81", 81N) optiques, électroniques ou opto-électroniques qui sont formées par sections de matériau transparent et/ou par des ouvertures dans la section d'enveloppe (82) dont le reste est fabriquée en matériau opaque.

10. Kit de dispositif d'analyse selon au moins une des revendications 7 à 9,
dans lequel deux des dispositifs de mesure (15, 17, 18) sont un dispositif de mesure photonique, de préférence un dispositif de mesure de luminescence (17C, 18C), et un dispositif de mesure d'indice de réfraction (17B, 18B), et dans lequel l'au moins un autre dispositif de mesure (15, 17, 18) est sélectionné parmi le groupe qui comprend au moins un dispositif de mesure de pH (17A, 18A), un dispositif de mesure de conductivité (15, 18D), un dispositif de mesure de nitrite (17AN, 18AN) et un dispositif de mesure (18E) pour la détection de la charge germinative,
- le dispositif de mesure de luminescence (17C, 18C), le dispositif de mesure d'indice de réfraction (17B, 18B), le dispositif de mesure de pH (17A, 18A) et le dispositif de mesure de nitrite (17AN, 18AN) présentent chacun un module de source lumineuse (17A, 17B, 17C, 17AN) et un module de détection (18A, 18B, 18C, 18AN) qui sont disposés dans le logement (2) des deux côtés des points de mesure correspondants (24, 25, 26, 27, 26N) de l'élément de réception d'échantillons (20) reçu dans l'appareil d'analyse (1),
- que l'appareil d'analyse (1) présente un dispositif de mesure de température (14) qui est connecté au module de traitement de données (13),
- le dispositif de mesure de conductivité (15, 18D) présente un générateur de fréquence (18D) avec des éléments de contact (15) qui sont en contact électrique avec les au moins deux bandes de contact (22) de l'élément de réception d'échantillons (20) en cas d'agencement de l'élément de réception d'échantillons (20) dans l'appareil d'analyse,
- le dispositif de mesure (18E) pour la détection de la charge germinative est au moins un capteur de gaz micro-électronique (18E) qui est en connexion avec l'espace de réception d'échantillons (31) par le biais d'une ligne de connexion.

11. Kit de dispositif d'analyse selon au moins une des revendications 8 à 10,
**caractérisé en ce que**
la section de bride (83) du dispositif d'insertion (8) dans un agencement d'analyse dans lequel le dispositif d'insertion (8) est inséré dans le logement (2) repose du côté externe sur un bord du logement (2) et encadre une plaque de recouvrement (4) dans laquelle l'ouverture d'insertion (9) est introduite, laquelle est étanchéifiée par une lèvre d'étanchéité (9') qui est maintenue par la plaque de recouvrement (4) dans la section de bride (83), dans lequel la plaque de recouvrement (4) est fixée de manière amovible dans la section de bride (83).

12. Kit de dispositif d'analyse selon la revendication 10 ou 11,
**caractérisé en ce que**
le dispositif d'insertion (8) présente des ponts de contact qui réalisent le contact des éléments de contact (15) de l'appareil d'analyse (1) avec les au moins deux bandes de contact (22) de l'élément de réception d'échantillons (20).

13. Kit de dispositif d'analyse selon au moins une des revendications 7 à 12,
**caractérisé en ce que**
l'appareil d'analyse (1) présente une source d'énergie, de préférence un accumulateur (11) qui est disposé dans le logement (2) et met à disposition l'alimentation en énergie du dispositif d'analyse opto-électronique (12), du module de traitement de données (13) et du dispositif d'affichage (3).

14. Kit de dispositif d'analyse selon au moins une des revendications 7 à 13,
**caractérisé en ce que**
- le dispositif d'affichage (3) est un dispositif d'affichage tactile (3) en tant qu'interface de commande,
- le module de traitement de données (13) présente une interface de communication externe (5) ou est connecté à celle-ci, dans lequel l'interface de communication externe (5) est une interface multibroche ou une interface radio.

15. Procédé d'analyse simultanée d'au moins trois paramètres chimiques-physiques différents d'un liquide utilisant un kit de dispositif d'analyse selon au moins une des revendications 7 à 14,
**comprenant les étapes**
- immersion de l'élément de réception d'échantillons (20) dans le liquide ou mise en contact de la surface de liquide avec une ouverture de l'élément de réception d'échantillons (20) qui est formée par les points de bord non connectés, et remplissage de l'espace de réception d'échantillons (31) de l'élément de réception d'échantillons (20) d'un échantillon liquide par l'action capillaire entre les doubles parois (30, 30') de l'élément de réception d'échantillons (20),
- insertion complète de l'élément de réception d'échantillons (20) dans l'appareil d'analyse (1),
- démarrage et réalisation d'au moins trois processus de mesure ou plus simultanément par les dispositifs de mesure (18A, B, C, D, E, AN) aux points de mesure (24, 25, 26, 27, 28, 26N),
- après la fin des processus de mesure, affichage des résultats de mesure sur le dispositif d'affichage (3).

16. Procédé selon la revendication 15,
**dans lequel**
- différents liquides pouvant être examinés sont déposés dans une base de données, lesquels sont mémorisés dans le module de traitement de données ou sur un support de stockage connecté à celui-ci, et avant le démarrage et la réalisation d'au moins trois processus de mesure ou plus simultanément par les dispositifs de mesure (18A, B, C, D, E, AN) aux points de mesure (24, 25, 26, 27, 28, 26N), sélection du liquide à examiner par une saisie d'utilisateur sur le dispositif d'affichage (3).

17. Procédé selon la revendication 15 ou 16,
**comprenant les étapes**
- détection de l'insertion complète de l'élément de réception d'échantillons (20) dans l'appareil d'analyse (1) automatiquement ou après saisie d'utilisateur et/ou
- affichage d'une demande de retrait de l'élément de réception d'échantillons (20) de l'appareil d'analyse (1) après la fin des processus de mesure sur le dispositif d'affichage (3), et/ou
- affichage des résultats de mesure sur le dispositif d'affichage (3) et mémorisation et/ou transmission des résultats de mesure après le retrait détecté de l'élément de réception d'échantillons (20) de l'appareil d'analyse (1).

18. Procédé selon la revendication 15 ou 16,
**comprenant les étapes**
- étalonnage de l'appareil d'analyse (1) pour les liquides pouvant être examinés qui sont déposés dans la base de données en utilisant des solutions d'étalonnage et/ou
- programmation de nouveaux liquides avec des paramètres chimiques-physiques connus avec l'appareil d'analyse (1) et ajout des liquides programmés dans la base de données.

19. Procédé selon la revendication 17 ou 18,
**dans lequel**
le liquide présente au moins une substance de marquage pouvant être détectée au moyen d'une analyse de luminescence et dans lequel un des points de mesure (24, 25, 26, 27, 28, 26N) est un point de mesure de luminescence (24).

20. Procédé selon la revendication 19,
**dans lequel**
le liquide à analyser est un fluide d'usinage métallique, notamment un lubrifiant caloporteur, de manière particulièrement préférée une émulsion de lubrifiant caloporteur, dans lequel au moins une première substance de marquage pouvant être détectée au moyen d'une analyse de luminescence est ajoutée au liquide en une concentration prédéterminée.

21. Procédé selon la revendication 20,
**dans lequel**
le liquide présente un additif renforçateur et au moins une seconde substance de marquage pouvant être détectée au moyen d'une analyse de luminescence est ajoutée au liquide en une concentration prédéterminée, laquelle se différencie de la première substance de marquage en ce qui concerne ses propriétés de luminescence.
